# EUROPEAN PATENT SPECIFICATION

(11) **EP 3 868 324 B1**
(45) Date of publication and mention of the grant of the patent: **14.10.2026**
(21) Application number: 21157271.4
(22) Date of filing: 16.02.2021
(51) Int. Cl.: A61B 34/10, A61B 34/20, A61B 34/30

(54) **DISPLAYING A VIRTUAL MODEL OF A PLANNED INSTRUMENT ATTACHMENT TO ENSURE CORRECT SELECTION OF PHYSICAL INSTRUMENT ATTACHMENT**
ANZEIGE EINES VIRTUELLEN MODELLS EINER GEPLANTEN INSTRUMENTENBEFESTIGUNG ZUR SICHERSTELLUNG DER KORREKTEN AUSWAHL DER PHYSISCHEN INSTRUMENTENBEFESTIGUNG
AFFICHAGE D'UN MODÈLE VIRTUEL D'UNE FIXATION D'INSTRUMENT PRÉVUE POUR ASSURER UNE SÉLECTION CORRECTE DE LA FIXATION D'INSTRUMENT PHYSIQUE

(30) Priority: 19.02.2020 US 202016794496
(43) Date of publication of application: 25.08.2021
(73) Proprietor: Globus Medical, Inc., Audubon, PA 19403 (US)
(72) Inventor: HEALY, Weston, CAMBRIDGE, MA 02141 (US); CALLOWAY, Thomas, PELHAM, NH 03076 (US); JOHNSON, Norbert, NORTH ANDOVER, MA 01845 (US)
(74) Representative: Morabito, Sara

(56) References cited:
- US-A1- 2018 116 732
- MARUYAMA KEISUKE ET AL: "CONCLUSION", vol. 15, no. 5, 24 January 2018 (2018-01-24), pages 551 - 556, XP055822632, ISSN: 2332-4252, Retrieved from the Internet <URL:http://academic.oup.com/ons/article-pdf/15/5/551/30213871/opx279.pdf> DOI: 10.1093/ons/opx279
- LIU HE ET AL: "Augmented Reality Based Navigation for Computer Assisted Hip Resurfacing: A Proof of Concept Study", ANNALS OF BIOMEDICAL ENGINEERING, SPRINGER US, NEW YORK, vol. 46, no. 10, 23 May 2018 (2018-05-23), pages 1595 - 1605, XP036596770, ISSN: 0090-6964, [retrieved on 20180523], DOI: 10.1007/S10439-018-2055-1
- KOREEDA YUTA ET AL: "Virtually transparent surgical instruments in endoscopic surgery with augmentation of obscured regions", INTERNATIONAL JOURNAL OF COMPUTER ASSISTED RADIOLOGY AND SURGERY, SPRINGER, DE, vol. 11, no. 10, 2 April 2016 (2016-04-02), pages 1927 - 1936, XP036062087, ISSN: 1861-6410, [retrieved on 20160402], DOI: 10.1007/S11548-016-1384-5

## Description

### FIELD

The present disclosure relates to medical devices and systems, and more particularly, computer assisted navigation in surgery using robotic surgical systems.

### BACKGROUND

Computer assisted navigation in surgery provides surgeons with enhanced visualization of surgical instruments with respect to radiographic images of the patient's anatomy. Navigated surgeries typically include components for tracking the position and orientation of surgical instruments via arrays of disks or spheres using a single near infrared ("NIR") stereo camera setup. In this scenario, there are three parameters jointly competing for optimization: (1) accuracy, (2) robustness and (3) ergonomics.

Navigated surgery procedures using existing navigation systems are prone to events triggering intermittent pauses while personnel and/or objects obstruct the ability of a tracking component to track poses of the patient, the robot, and surgical instruments. There is a need to improve the tracking performance of navigation systems.

Maruyama et al, "Smart glasses for Neurosurgical Navigation by Augmented Reality", Operative Neurosurgery, 2018, pp. 1-6 discloses smart glasses which enabled AR-based neurosurgical navigation in a hands-free fashion.

Liu et al., "Augmented Reality Based Navigation for Computer Assisted Hip Resurfacing: A Proof of Concept Study", Annals of Biomedical Engineering, 2018, Vol. 46, No. 10, pp. 1595-1606 discloses an augmented-reality (AR) based navigation system for hip resurfacing.

### SUMMARY

The invention is defined in the appended claims.

Various embodiments disclosed herein are directed to improvements in computer assisted navigation during surgery. One or more extended reality ("XR") headsets can be equipped with tracking cameras that provide tracking information to a camera tracking system for combining with tracking information from other tracking cameras which may be part of another XR headset, an auxiliary tracking bar, or other equipment. Through various pose chaining operations disclosed herein the camera tracking system may be able to track tools and other objects with greater robustness and through a wide range of motion.

In some embodiments, a surgical system is provided. The surgical system includes an extended reality ("XR") headset and an XR headset controller. The XR headset is configured to be worn by a user during a surgical procedure and includes a see-through display screen configured to display a virtual model of a planned instrument attachment and to allow at least a portion of a real-world scene to pass therethrough for viewing by the user. The real-world scene includes an instrument. The XR headset controller is configured to generate a shape and a pose of the virtual model of the planned instrument attachment based on predetermined information associated with the planned instrument attachment and based on a pose of the instrument relative to the XR headset.

In other embodiments, a surgical system is provided. The surgical system includes an extended reality ("XR") headset and an XR headset controller. The XR headset is configured to be worn by a user during a surgical procedure and includes a see-through display screen configured to display a virtual model of a planned instrument attachment and to allow at least a portion of a real-world scene to pass therethrough for viewing by the user. The real-world scene includes a physical instrument attachment. The XR headset controller is configured to generate a shape and a pose of the virtual model of the planned instrument attachment based on predetermined information of the planned instrument attachment and based on a pose of the physical instrument attachment relative to the XR headset.

Related methods by a surgical system and related robotic surgical systems are disclosed.

Other surgical systems, method, and computer program products according to embodiments will be or become apparent to one with skill in the art upon review of the following drawings and detailed description. It is intended that all such surgical systems, method, and computer program products be included within this description, be within the scope of the present disclosure, and the invention is protected by the accompanying claims. Moreover, it is intended that all embodiments disclosed herein can be implemented separately or combined in any way and/or combination.

### BRIEF DESCRIPTION OF THE DRAWINGS

The accompanying drawings, which are included to provide a further understanding of the disclosure and are incorporated in a constitute a part of this application, illustrate certain non-limiting embodiments of inventive concepts. In the drawings:
Figure 1 illustrates an embodiment of a surgical system according to some embodiments of the present disclosure;
Figure 2 illustrates a surgical robot component of the surgical system of Figure 1 according to some embodiments of the present disclosure;
Figure 3A illustrates a camera tracking system component of the surgical system of Figure 1 according to some embodiments of the present disclosure;
Figures 3B and 3C illustrate a front view and isometric view of another camera tracking system component which may be used with the surgical system of Figure 1 according to some embodiments of the present disclosure;
Figure 4 illustrates an embodiment of an end effector that is connectable to a robot arm and configured according to some embodiments of the present disclosure;
Figure 5 illustrates a medical operation in which a surgical robot and a camera system are disposed around a patient;
Figure 6 illustrates a block diagram view of the components of the surgical system of Figure 5 being used for a medical operation;
Figure 7 illustrates various display screens that may be displayed on the display of Figures 5 and 6 when using a navigation function of the surgical system;
Figure 8 illustrates a block diagram of some electrical components of a surgical robot according to some embodiments of the present disclosure;
Figure 9 illustrates a block diagram of components of a surgical system that includes imaging devices connected to a computer platform which can be operationally connected to a camera tracking system and/or surgical robot according to some embodiments of the present disclosure;
Figure 10 illustrates an embodiment of a C-Arm imaging device that can be used in combination with the surgical robot in accordance with some embodiments of the present disclosure;
Figure 11 illustrates an embodiment of an O-Arm imaging device that can be used in combination with the surgical robot in accordance with some embodiments of the present disclosure;
Figure 12 illustrates a block diagram view of the components of a surgical system that includes a pair of XR headsets and an auxiliary tracking bar which operate in accordance with some embodiments of the present disclosure;
Figure 13 illustrates an XR headset which is configured in accordance with some embodiments of the present disclosure;
Figure 14 illustrates electrical components of the XR headset that can be operatively connected to a computer platform, imaging device(s), and/or a surgical robot in accordance with some embodiments of the present disclosure;
Figure 15 illustrates a block diagram showing arrangement of optical components of the XR headset in accordance with some embodiments of the present disclosure;
Figure 16 illustrates an example view through the display screen of an XR headset for providing navigation assistance to manipulate a surgical tool during a medical procedure in accordance with some embodiments of the present disclosure;
Figure 17 illustrates an example configuration of an auxiliary tracking bar having two pairs of stereo cameras configured in accordance with some embodiments of the present disclosure;
Figures 18A-C illustrate examples of a view through the display screen of an XR headset in which a virtual model or information associated with an instrument attachment is displayed in accordance with some embodiments of the present disclosure;
Figures 19A-C illustrate examples of a view through the display screen of an XR headset in which a virtual model of an instrument attachment is displayed at a pose based on the instrument in accordance with some embodiments of the present disclosure;
Figures 20A-C illustrate examples of a view through the display screen of an XR headset in which a virtual model of an instrument attachment at a pose based on the instrument matches a real-world instrument attachment in accordance with some embodiments of the present disclosure;
Figures 21A-C illustrate examples of a view through the display screen of an XR headset in which a virtual model of an instrument attachment at a pose based on the instrument does not match a real-world instrument attachment in accordance with some embodiments of the present disclosure; and
Figures 22-23 illustrate flowcharts that are examples of processes performed by a surgical system in accordance with some embodiments of the present disclosure.

### DETAILED DESCRIPTION

Inventive concepts will now be described more fully hereinafter with reference to the accompanying drawings, in which examples of embodiments of inventive concepts are shown. Inventive concepts may, however, be embodied in many different forms and should not be construed as limited to the embodiments set forth herein. Rather, these embodiments are provided so that this disclosure will be thorough and complete, and will fully convey the scope of various present inventive concepts to those skilled in the art. It should also be noted that these embodiments are not mutually exclusive. Components from one embodiment may be tacitly assumed to be present or used in another embodiment.

Various embodiments disclosed herein are directed to improvements in computer assisted navigation during surgery. An extended reality ("XR") headset is operatively connected to the surgical system and configured to provide an interactive environment through which a surgeon, assistant, and/or other personnel can view and select among patient images, view and select among computer generated surgery navigation information, and/or control surgical equipment in the operating room. As will be explained below, the XR headset may be configured to augment a real-world scene with computer generated XR images. The XR headset may be configured to provide an augmented reality ("AR") viewing environment by displaying the computer generated XR images on a see-through display screen that allows light from the real-world scene to pass therethrough for combined viewing by the user. Alternatively, the XR headset may be configured to provide a virtual reality ("VR") viewing environment by preventing or substantially preventing light from the real-world scene from being directly viewed by the user while the user is viewing the computer generated AR images on a display screen. An XR headset can be configured to provide both AR and VR viewing environments. In one embodiment, both AR and VR viewing environments are provided by lateral bands of substantially differing opacity arranged between the see-through display screen and the real-world scene, so that a VR viewing environment is provided for XR images aligned with a high opacity band and an AR viewing environment is provided for XR images aligned with the low opacity band. In another embodiment, both AR and VR viewing environments are provided by computer adjustable control of an opacity filter that variably constrains how much light from the real-world scene passes through a see-through display screen for combining with the XR images viewed by the user. Thus, the XR headset can also be referred to as an AR headset or a VR headset.

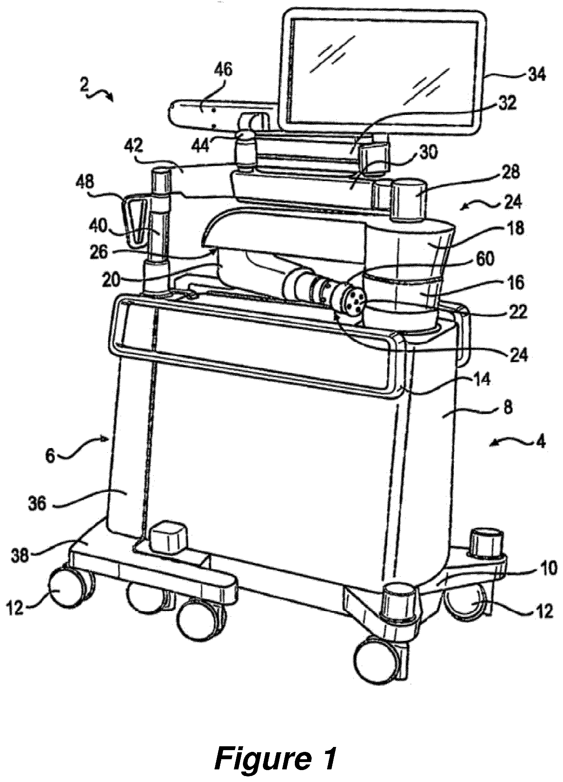

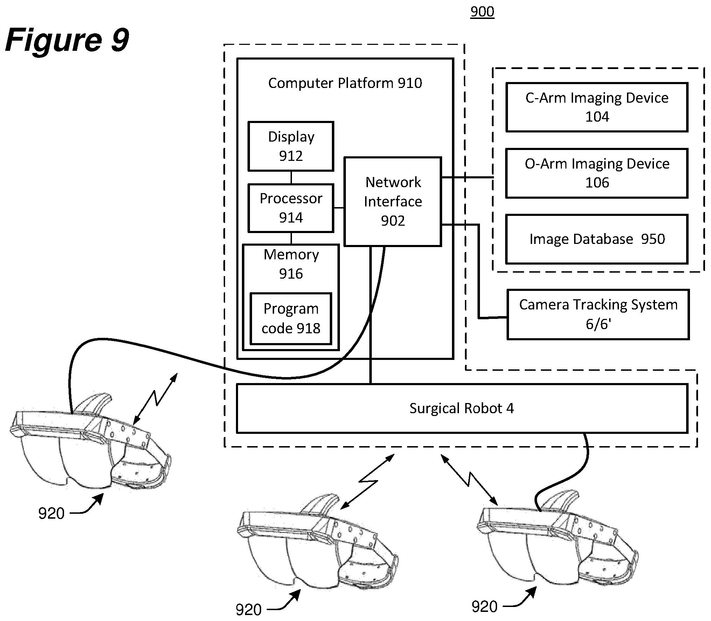

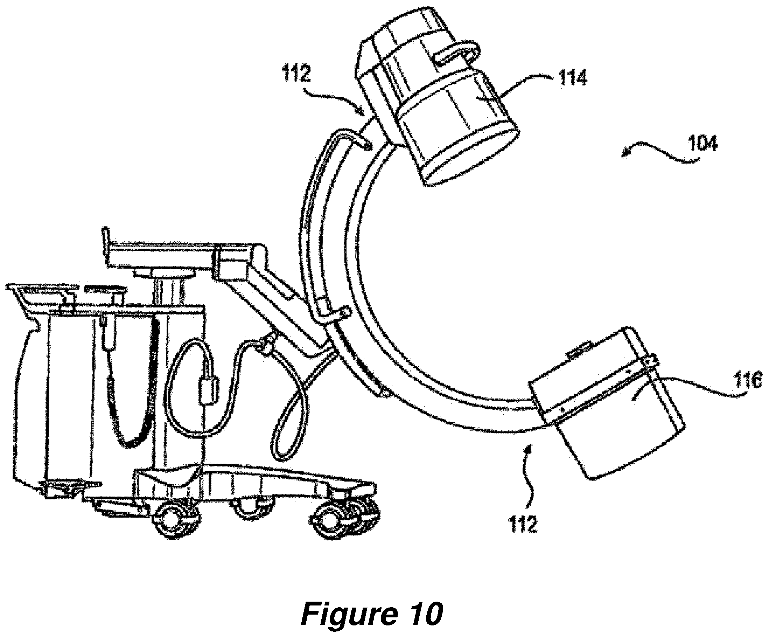

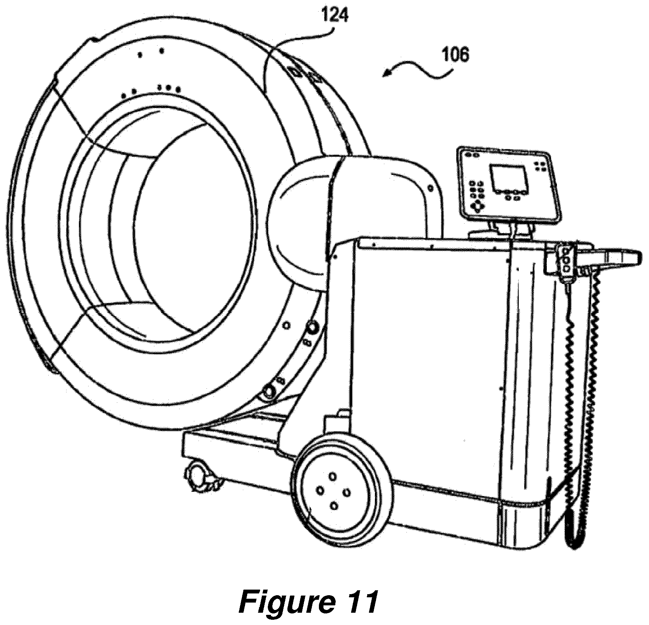

Figure 1 illustrates an embodiment of a surgical system 2 according to some embodiments of the present disclosure. Prior to performance of an orthopedic or other surgical procedure, a three-dimensional ("3D") image scan may be taken of a planned surgical area of a patient using, e.g., the C-Arm imaging device 104 of Fig. 10 or O-Arm imaging device 106 of Fig. 11, or from another medical imaging device such as a computed tomography ("CT") image or MRI. This scan can be taken pre-operatively (e.g. few weeks before procedure, most common) or intra-operatively. However, any known 3D or 2D image scan may be used in accordance with various embodiments of the surgical system 2. The image scan is sent to a computer platform in communication with the surgical system 2, such as the computer platform 910 of the surgical system 900 (Fig. 9) which may include the camera tracking system component 6, the surgical robot 4 (e.g., robot 2 in Fig. 1), imaging devices (e.g., C-Arm 104, O-Arm 106, etc.), and an image database 950 for storing image scans of patients. A surgeon reviewing the image scan(s) on a display device of the computer platform 910 (Fig. 9) generates a surgical plan defining a target pose for a surgical tool to be used during a surgical procedure on an anatomical structure of the patient. Example surgical tools, also referred to as tools, can include, without limitation, drills, screw drivers, retractors, and implants such as a screws, spacers, interbody fusion devices, plates, rods, etc. In some embodiments, the surgical plan defining the target plane is planned on the 3D image scan displayed on a display device.

As used herein, the term "pose" refers to the position and/or the rotational angle of one object (e.g., dynamic reference array, end effector, surgical tool, anatomical structure, etc.) relative to another object and/or to a defined coordinate system. A pose may therefore be defined based on only the multidimensional position of one object relative to another object and/or to a defined coordinate system, only on the multidimensional rotational angles of the object relative to another object and/or to a defined coordinate system, or on a combination of the multidimensional position and the multidimensional rotational angles. The term "pose" therefore is used to refer to position, rotational angle, or combination thereof.

The surgical system 2 of Figure 1 can assist surgeons during medical procedures by, for example, holding tools, aligning tools, using tools, guiding tools, and/or positioning tools for use. In some embodiments, surgical system 2 includes a surgical robot 4 and a camera tracking system component 6. The ability to mechanically couple surgical robot 4 and camera tracking system component 6 can allow for surgical system 2 to maneuver and move as a single unit, and allow surgical system 2 to have a small footprint in an area, allow easier movement through narrow passages and around turns, and allow storage within a smaller area.

A surgical procedure may begin with the surgical system 2 moving from medical storage to a medical procedure room. The surgical system 2 may be maneuvered through doorways, halls, and elevators to reach a medical procedure room. Within the room, the surgical system 2 may be physically separated into two separate and distinct systems, the surgical robot 4 and the camera tracking system component 6. Surgical robot 4 may be positioned adjacent the patient at any suitable location to properly assist medical personnel. Camera tracking system component 6 may be positioned at the base of the patient, at the patient shoulders, or any other location suitable to track the present pose and movement of the pose of tracks portions of the surgical robot 4 and the patient. Surgical robot 4 and camera tracking system component 6 may be powered by an onboard power source and/or plugged into an external wall outlet.

Surgical robot 4 may be used to assist a surgeon by holding and/or using tools during a medical procedure. To properly utilize and hold tools, surgical robot 4 may rely on a plurality of motors, computers, and/or actuators to function properly. Illustrated in Figure 1, robot body 8 may act as the structure in which the plurality of motors, computers, and/or actuators may be secured within surgical robot 4. Robot body 8 may also provide support for robot telescoping support arm 16. The size of robot body 8 may provide a solid platform supporting attached components, and may house, conceal, and protect the plurality of motors, computers, and/or actuators that may operate attached components.

Robot base 10 may act as a lower support for surgical robot 4. In some embodiments, robot base 10 may support robot body 8 and may attach robot body 8 to a plurality of powered wheels 12. This attachment to wheels may allow robot body 8 to move in space efficiently. Robot base 10 may run the length and width of robot body 8. Robot base 10 may be about 5.08 cm (2 inches) to about 25.4 cm (10 inches) tall. Robot base 10 may cover, protect, and support powered wheels 12.

In some embodiments, as illustrated in Figure 1, at least one powered wheel 12 may be attached to robot base 10. Powered wheels 12 may attach to robot base 10 at any location. Each individual powered wheel 12 may rotate about a vertical axis in any direction. A motor may be disposed above, within, or adjacent to powered wheel 12. This motor may allow for surgical system 2 to maneuver into any location and stabilize and/or level surgical system 2. A rod, located within or adjacent to powered wheel 12, may be pressed into a surface by the motor. The rod, not pictured, may be made of any suitable metal to lift surgical system 2. The rod may lift powered wheel 10, which may lift surgical system 2, to any height required to level or otherwise fix the orientation of the surgical system 2 in relation to a patient. The weight of surgical system 2, supported through small contact areas by the rod on each wheel, prevents surgical system 2 from moving during a medical procedure. This rigid positioning may prevent objects and/or people from moving surgical system 2 by accident.

Moving surgical system 2 may be facilitated using robot railing 14. Robot railing 14 provides a person with the ability to move surgical system 2 without grasping robot body 8. As illustrated in Figure 1, robot railing 14 may run the length of robot body 8, shorter than robot body 8, and/or may run longer the length of robot body 8. Robot railing 14 may further provide protection to robot body 8, preventing objects and or personnel from touching, hitting, or bumping into robot body 8.

Robot body 8 may provide support for a Selective Compliance Articulated Robot Arm, hereafter referred to as a "SCARA." A SCARA 24 may be beneficial to use within the surgical system 2 due to the repeatability and compactness of the robotic arm. The compactness of a SCARA may provide additional space within a medical procedure, which may allow medical professionals to perform medical procedures free of excess clutter and confining areas. SCARA 24 may comprise robot telescoping support 16, robot support arm 18, and/or robot arm 20. Robot telescoping support 16 may be disposed along robot body 8. As illustrated in Figure 1, robot telescoping support 16 may provide support for the SCARA 24 and display 34. In some embodiments, robot telescoping support 16 may extend and contract in a vertical direction. The body of robot telescoping support 16 may be any width and/or height configured to support the stress and weight placed upon it.

In some embodiments, medical personnel may move SCARA 24 through a command submitted by the medical personnel. The command may originate from input received on display 34, a tablet, and/or an XR headset (e.g., headset 920 in Fig. 9) as will be explained in further detail below. The XR headset may eliminate the need for medical personnel to refer to any other display such as the display 34 or a tablet, which enables the SCARA 24 to be configured without the display 34 and/or the tablet. The command may be generated by the depression of a switch and/or the depression of a plurality of switches, and/or may be generated based on a hand gesture command and/or voice command that is sensed by the XR headset as will be explained in further detail below.

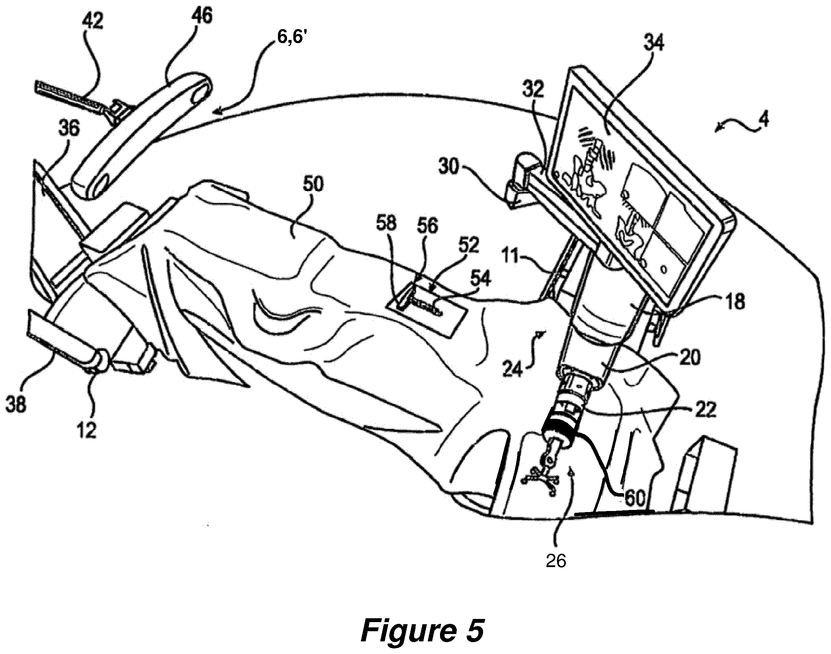

As shown in Figure 5, an activation assembly 60 may include a switch and/or a plurality of switches. The activation assembly 60 may be operable to transmit a move command to the SCARA 24 allowing an operator to manually manipulate the SCARA 24. When the switch, or plurality of switches, is depressed the medical personnel may have the ability to move SCARA 24 through applied hand movements. Alternatively or additionally, an operator may control movement of the SCARA 24 through hand gesture commands and/or voice commands that are sensed by the XR headset as will be explained in further detail below. Additionally, when the SCARA 24 is not receiving a command to move, the SCARA 24 may lock in place to prevent accidental movement by personnel and/or other objects. By locking in place, the SCARA 24 provides a solid platform through which the end effector 26 can guide a surgical tool during a medical procedure.

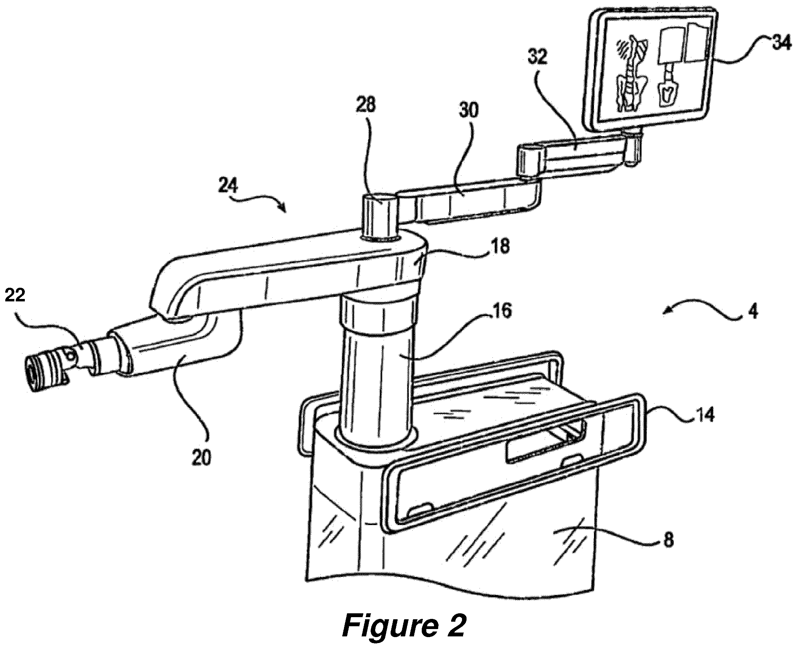

Robot support arm 18 can be connected to robot telescoping support 16 by various mechanisms. In some embodiments, best seen in Figures 1 and 2, robot support arm 18 rotates in any direction in regard to robot telescoping support 16. Robot support arm 18 may rotate three hundred and sixty degrees around robot telescoping support 16. Robot arm 20 may connect to robot support arm 18 at any suitable location and by various mechanisms that enable rotation in any direction relative to robot support arm 18. In one embodiment, the robot arm 20 can rotate three hundred and sixty degrees relative to the robot support arm 18. This free rotation allows an operator to position robot arm 20 according to a surgical plan.

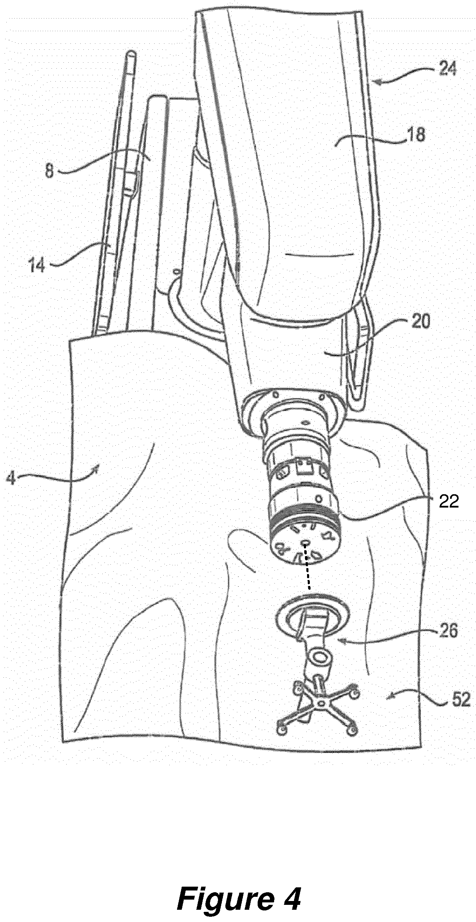

The end effector 26 shown in Figures 4 and 5 may attach to robot arm 20 in any suitable location. The end effector 26 can be configured to attach to an end effector coupler 22 of the robot arm 20 positioned by the surgical robot 4. The example end effector 26 includes a tubular guide that guides movement of an inserted surgical tool relative to an anatomical structure on which a surgical procedure is to be performed.

In some embodiments, a dynamic reference array 52 is attached to the end effector 26. Dynamic reference arrays, also referred to as "DRAs" herein, are rigid bodies which may be disposed on an anatomical structure (e.g., bone) of a patient, one or more XR headsets being worn by personnel in the operating room, the end effector, the surgical robot, a surgical tool in a navigated surgical procedure. The computer platform 910 in combination with the camera tracking system component 6 or other 3D localization system are configured to track in real-time the pose (e.g., positions and rotational orientations) of the DRA. The DRA can include fiducials, such as the illustrated arrangement of balls. This tracking of 3D coordinates of the DRA can allow the surgical system 2 to determine the pose of the DRA in any multidimensional space in relation to the target anatomical structure of the patient 50 in Figure 5.

As illustrated in Figure 1, a light indicator 28 may be positioned on top of the SCARA 24. Light indicator 28 may illuminate as any type of light to indicate "conditions" in which surgical system 2 is currently operating. In some embodiments, the light may be produced by LED bulbs, which may form a ring around light indicator 28. Light indicator 28 may comprise a fully permeable material that can let light shine through the entirety of light indicator 28. Light indicator 28 may be attached to lower display support 30. Lower display support 30, as illustrated in Figure 2 may allow an operator to maneuver display 34 to any suitable location. Lower display support 30 may attach to light indicator 28 by any suitable mechanism. In some embodiments, lower display support 30 may rotate about light indicator 28 or be rigidly attached thereto. Upper display support 32 may attach to lower display support 30 by any suitable mechanism.

In some embodiments, a tablet may be used in conjunction with display 34 and/or without display 34. The tablet may be disposed on upper display support 32, in place of display 34, and may be removable from upper display support 32 during a medical operation. In addition the tablet may communicate with display 34. The tablet may be able to connect to surgical robot 4 by any suitable wireless and/or wired connection. In some embodiments, the tablet may be able to program and/or control surgical system 2 during a medical operation. When controlling surgical system 2 with the tablet, all input and output commands may be duplicated on display 34. The use of a tablet may allow an operator to manipulate surgical robot 4 without having to move around patient 50 and/or to surgical robot 4.

As will be explained below, in some embodiments a surgeon and/or other personnel can wear XR headsets that may be used in conjunction with display 34 and/or a tablet or the XR head(s) may eliminate the need for use of the display 34 and/or tablet.

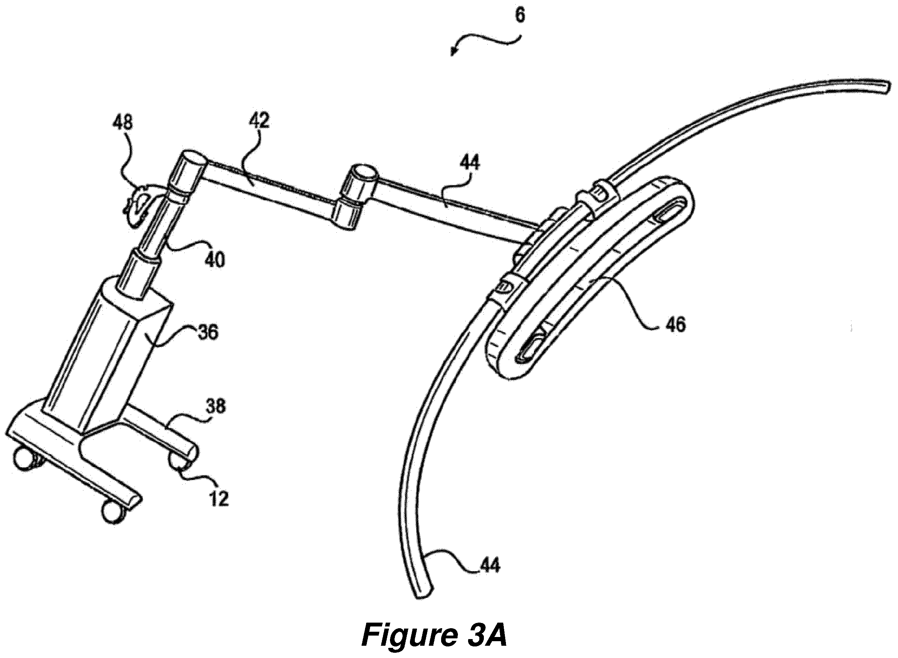

As illustrated in Figures 3A and 5, camera tracking system component 6 works in conjunction with surgical robot 4 through wired or wireless communication networks. Referring to Figures 1, 3 and 5, camera tracking system component 6 can include some similar components to the surgical robot 4. For example, camera body 36 may provide the functionality found in robot body 8. Robot body 8 may provide an auxiliary tracking bar upon which cameras 46 are mounted. The structure within robot body 8 may also provide support for the electronics, communication devices, and power supplies used to operate camera tracking system component 6. Camera body 36 may be made of the same material as robot body 8. Camera tracking system component 6 may communicate directly to an XR headset, tablet and/or display 34 by a wireless and/or wired network to enable the XR headset, tablet and/or display 34 to control the functions of camera tracking system component 6.

Camera body 36 is supported by camera base 38. Camera base 38 may function as robot base 10. In the embodiment of Figure 1, camera base 38 may be wider than robot base 10. The width of camera base 38 may allow for camera tracking system component 6 to connect with surgical robot 4. As illustrated in Figure 1, the width of camera base 38 may be large enough to fit outside robot base 10. When camera tracking system component 6 and surgical robot 4 are connected, the additional width of camera base 38 may allow surgical system 2 additional maneuverability and support for surgical system 2.

As with robot base 10, a plurality of powered wheels 12 may attach to camera base 38. Powered wheel 12 may allow camera tracking system component 6 to stabilize and level or set fixed orientation in regards to patient 50, similar to the operation of robot base 10 and powered wheels 12. This stabilization may prevent camera tracking system component 6 from moving during a medical procedure and may keep cameras 46 on the auxiliary tracking bar from losing track of a DRA connected to an XR headset and/or the surgical robot 4, and/or losing track of one or more DRAs 52 connected to an anatomical structure 54 and/or tool 58 within a designated area 56 as shown in Figures 3A and 5. This stability and maintenance of tracking enhances the ability of surgical robot 4 to operate effectively with camera tracking system component 6. Additionally, the wide camera base 38 may provide additional support to camera tracking system component 6. Specifically, a wide camera base 38 may prevent camera tracking system component 6 from tipping over when cameras 46 is disposed over a patient, as illustrated in Figures 3A and 5.

Camera telescoping support 40 may support cameras 46 on the auxiliary tracking bar. In some embodiments, telescoping support 40 moves cameras 46 higher or lower in the vertical direction. Camera handle 48 may be attached to camera telescoping support 40 at any suitable location and configured to allow an operator to move camera tracking system component 6 into a planned position before a medical operation. In some embodiments, camera handle 48 is used to lower and raise camera telescoping support 40. Camera handle 48 may perform the raising and lowering of camera telescoping support 40 through the depression of a button, switch, lever, and/or any combination thereof.

Lower camera support arm 42 may attach to camera telescoping support 40 at any suitable location, in embodiments, as illustrated in Figure 1, lower camera support arm 42 may rotate three hundred and sixty degrees around telescoping support 40. This free rotation may allow an operator to position cameras 46 in any suitable location. Lower camera support arm 42 may connect to telescoping support 40 by any suitable mechanism. Lower camera support arm 42 may be used to provide support for cameras 46. Cameras 46 may be attached to lower camera support arm 42 by any suitable mechanism. Cameras 46 may pivot in any direction at the attachment area between cameras 46 and lower camera support arm 42. In embodiments a curved rail 44 may be disposed on lower camera support arm 42.

Curved rail 44 may be disposed at any suitable location on lower camera support arm 42. As illustrated in Figure 3A, curved rail 44 may attach to lower camera support arm 42 by any suitable mechanism. Curved rail 44 may be of any suitable shape, a suitable shape may be a crescent, circular, oval, elliptical, and/or any combination thereof. Cameras 46 may be moveably disposed along curved rail 44. Cameras 46 may attach to curved rail 44 by, for example, rollers, brackets, braces, motors, and/or any combination thereof. Motors and rollers, not illustrated, may be used to move cameras 46 along curved rail 44. As illustrated in Figure 3A, during a medical procedure, if an object prevents cameras 46 from viewing one or more DRAs being tracked, the motors may responsively move cameras 46 along curved rail 44. This motorized movement may allow cameras 46 to move to a new position that is no longer obstructed by the object without moving camera tracking system component 6. While cameras 46 is obstructed from viewing one or more tracked DRAs, camera tracking system component 6 may send a stop signal to a surgical robot 4, XR headset, display 34, and/or a tablet. The stop signal may prevent SCARA 24 from moving until cameras 46 has reacquired tracked DRAs 52 and/or can warn an operator wearing the XR headset and/or viewing the display 34 and/or the tablet. This SCARA 24 can be configured to respond to receipt of a stop signal by stopping further movement of the base and/or end effector coupler 22 until the camera tracking system can resume tracking of DRAs.

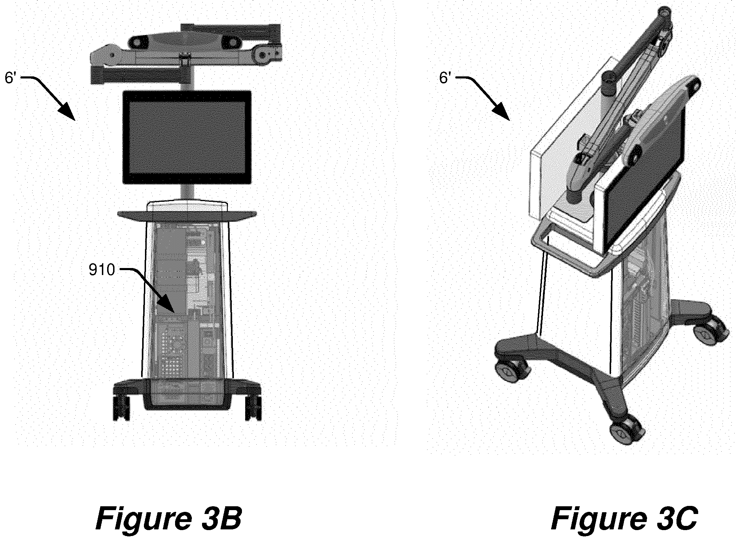

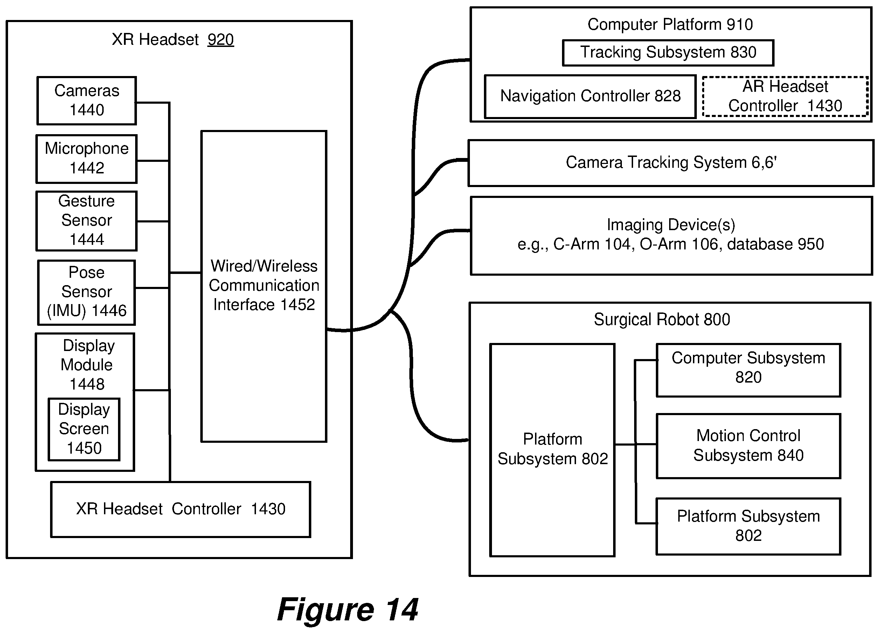

Figures 3B and 3C illustrate a front view and isometric view of another camera tracking system component 6' which may be used with the surgical system of Figure 1 or may be used independent of a surgical robot. For example, the camera tracking system component 6' may be used for providing navigated surgery without use of robotic guidance. One of the differences between the camera tracking system component 6' of Figures 3B and 3C and the camera tracking system component 6 of Figure 3A, is that the camera tracking system component 6' of Figures 3B and 3C includes a housing that transports the computer platform 910. The computer platform 910 can be configured to perform camera tracking operations to track DRAs, perform navigated surgery operations that provide surgical navigation information to a display device, e.g., XR headset and/or other display device, and perform other computational operations disclosed herein. The computer platform 910 can therefore include a navigation computer, such as one or more of the navigation computers of Figures 14.

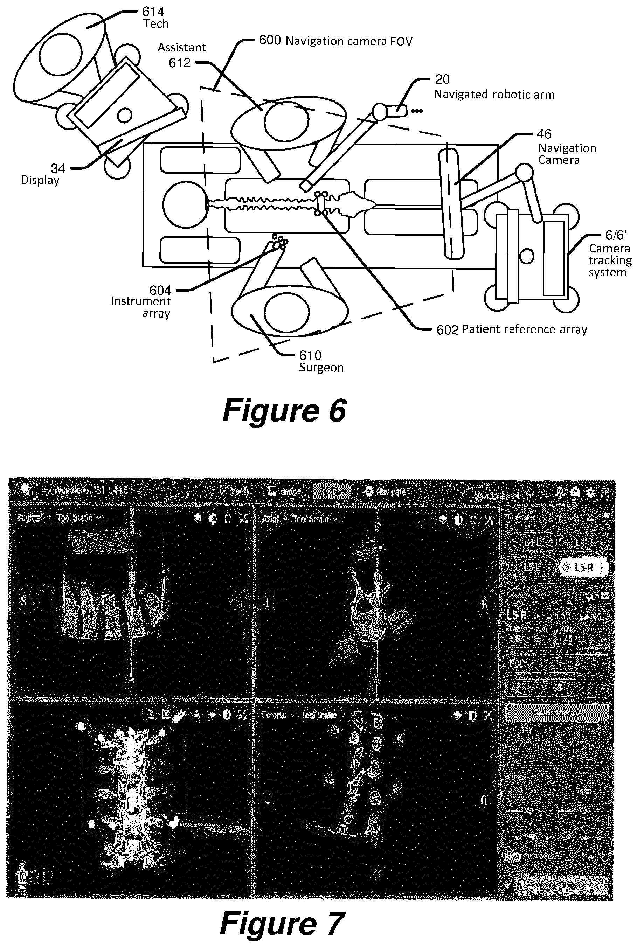

Figure 6 illustrates a block diagram view of the components of the surgical system of Figure 5 used for the medical operation. Referring to Figure 6, the navigation cameras 46 on the auxiliary tracking bar has a navigation field-of-view 600 in which the pose (e.g., position and orientation) of the reference array 602 attached to the patient, the reference array 604 attached to the surgical instrument, and the robot arm 20 are tracked. The navigation cameras 46 may be part of the camera tracking system component 6' of Figures 3B and 3C, which includes the computer platform 910 configured to perform the operations described below. The reference arrays enable tracking by reflecting light in known patterns, which are decoded to determine their respective poses by the tracking subsystem of the surgical robot 4. If the line-of-sight between the patient reference array 602 and the navigation cameras 46 in the auxiliary tracking bar is blocked (for example, by a medical personnel, instrument, etc.), further navigation of the surgical instrument may not be able to be performed and a responsive notification may temporarily halt further movement of the robot arm 20 and surgical robot 4, display a warning on the display 34, and/or provide an audible warning to medical personnel. The display 34 is accessible to the surgeon 610 and assistant 612 but viewing requires a head to be turned away from the patient and for eye focus to be changed to a different distance and location. The navigation software may be controlled by a tech personnel 614 based on vocal instructions from the surgeon.

Figure 7 illustrates various display screens that may be displayed on the display 34 of Figures 5 and 6 by the surgical robot 4 when using a navigation function of the surgical system 2. The display screens can include, without limitation, patient radiographs with overlaid graphical representations of models of instruments that are positioned in the display screens relative to the anatomical structure based on a developed surgical plan and/or based on poses of tracked reference arrays, various user selectable menus for controlling different stages of the surgical procedure and dimension parameters of a virtually projected implant (e.g. length, width, and/or diameter).

For navigated surgery, various processing components (e.g., computer platform 910) and associated software described below are provided that enable pre-operatively planning of a surgical procedure, e.g., implant placement, and electronic transfer of the plan to computer platform 910 to provide navigation information to one or more users during the planned surgical procedure.

For robotic navigation, various processing components (e.g., computer platform 910) and associated software described below are provided that enable pre-operatively planning of a surgical procedure, e.g., implant placement, and electronic transfer of the plan to the surgical robot 4. The surgical robot 4 uses the plan to guide the robot arm 20 and connected end effector 26 to provide a target pose for a surgical tool relative to a patient anatomical structure for a step of the planned surgical procedure.

Various embodiments below are directed to using one or more XR headsets that can be worn by the surgeon 610, the assistant 612, and/or other medical personnel to provide an improved user interface for receiving information from and/or providing control commands to the surgical robot, the camera tracking system component 6/6', and/or other medical equipment in the operating room.

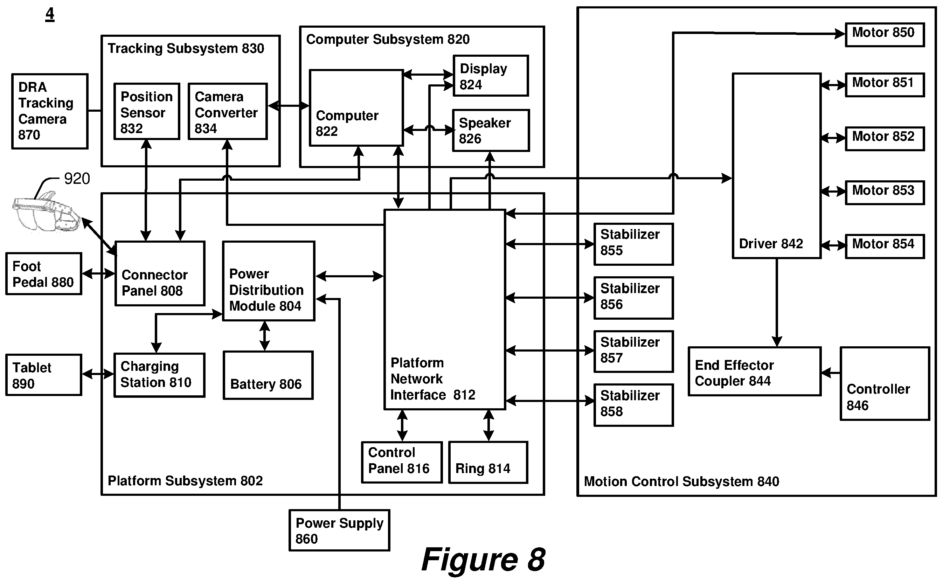

Figure 8 illustrates a block diagram of some electrical components of the surgical robot 4 according to some embodiments of the present disclosure. Referring to Figure 8, a load cell (not shown) may be configured to track force applied to end effector coupler 22. In some embodiments the load cell may communicate with a plurality of motors 850, 851, 852, 853, and/or 854. As load cell senses force, information as to the amount of force applied may be distributed from a switch array and/or a plurality of switch arrays to a controller 846. Controller 846 may take the force information from load cell and process it with a switch algorithm. The switch algorithm is used by the controller 846 to control a motor driver 842. The motor driver 842 controls operation of one or more of the motors 850, 851, 852, 853, and 854. Motor driver 842 may direct a specific motor to produce, for example, an equal amount of force measured by load cell through the motor. In some embodiments, the force produced may come from a plurality of motors, e.g., 850-854, as directed by controller 846. Additionally, motor driver 842 may receive input from controller 846. Controller 846 may receive information from load cell as to the direction of force sensed by load cell. Controller 846 may process this information using a motion controller algorithm. The algorithm may be used to provide information to specific motor drivers 842. To replicate the direction of force, controller 846 may activate and/or deactivate certain motor drivers 842. Controller 846 may control one or more motors, e.g. one or more of 850-854, to induce motion of end effector 26 in the direction of force sensed by load cell. This force-controlled motion may allow an operator to move SCARA 24 and end effector 26 effortlessly and/or with very little resistance. Movement of end effector 26 can be performed to position end effector 26 in any suitable pose (i.e., location and angular orientation relative to defined three-dimensional ("3D") orthogonal reference axes) for use by medical personnel.

Activation assembly 60, best illustrated in Figure 5, may form of a bracelet that wraps around end effector coupler 22. The activation assembly 60 may be located on any part of SCARA 24, any part of end effector coupler 22, may be worn by medical personnel (and communicate wirelessly), and/or any combination thereof. Activation assembly 60 may comprise of a primary button and a secondary button.

Depressing primary button may allow an operator to move SCARA 24 and end effector coupler 22. According to one embodiment, once set in place, SCARA 24 and end effector coupler 22 may not move until an operator programs surgical robot 4 to move SCARA 24 and end effector coupler 22, or is moved using primary button. In some examples, it may require the depression of at least two non-adjacent primary activation switches before SCARA 24 and end effector coupler 22 will respond to operator commands. Depression of at least two primary activation switches may prevent the accidental movement of SCARA 24 and end effector coupler 22 during a medical procedure.

Activated by primary button, load cell may measure the force magnitude and/or direction exerted upon end effector coupler 22 by an operator, i.e. medical personnel. This information may be transferred to one or more motors, e.g. one or more of 850-854, within SCARA 24 that may be used to move SCARA 24 and end effector coupler 22. Information as to the magnitude and direction of force measured by load cell may cause the one or more motors, e.g. one or more of 850-854, to move SCARA 24 and end effector coupler 22 in the same direction as sensed by the load cell. This force-controlled movement may allow the operator to move SCARA 24 and end effector coupler 22 easily and without large amounts of exertion due to the motors moving SCARA 24 and end effector coupler 22 at the same time the operator is moving SCARA 24 and end effector coupler 22.

In some examples, a secondary button may be used by an operator as a "selection" device. During a medical operation, surgical robot 4 may notify medical personnel to certain conditions by the XR headset(s) 920, display 34 and/or light indicator 28. The XR headset(s) 920 are each configured to display images on a see-through display screen to form an extended reality image that is overlaid on real-world objects viewable through the see-through display screen. Medical personnel may be prompted by surgical robot 4 to select a function, mode, and/or asses the condition of surgical system 2. Depressing secondary button a single time may activate certain functions, modes, and/or acknowledge information communicated to medical personnel through the XR headset(s) 920, display 34 and/or light indicator 28. Additionally, depressing the secondary button multiple times in rapid succession may activate additional functions, modes, and/or select information communicated to medical personnel through the XR headset(s) 920, display 34 and/or light indicator 28.

With further reference to Figure 8, electrical components of the surgical robot 4 include platform subsystem 802, computer subsystem 820, motion control subsystem 840, and tracking subsystem 830. Platform subsystem 802 includes battery 806, power distribution module 804, connector panel 808, and charging station 810. Computer subsystem 820 includes computer 822, display 824, and speaker 826. Motion control subsystem 840 includes driver circuit 842, motors 850, 851, 852, 853, 854, stabilizers 855, 856, 857, 858, end effector connector 844, and controller 846. Tracking subsystem 830 includes position sensor 832 and camera converter 834. Surgical robot 4 may also include a removable foot pedal 880 and removable tablet computer 890.

Input power is supplied to surgical robot 4 via a power source which may be provided to power distribution module 804. Power distribution module 804 receives input power and is configured to generate different power supply voltages that are provided to other modules, components, and subsystems of surgical robot 4. Power distribution module 804 may be configured to provide different voltage supplies to connector panel 808, which may be provided to other components such as computer 822, display 824, speaker 826, driver 842 to, for example, power motors 850-854 and end effector coupler 844, and provided to camera converter 834 and other components for surgical robot 4. Power distribution module 804 may also be connected to battery 806, which serves as temporary power source in the event that power distribution module 804 does not receive power from an input power. At other times, power distribution module 804 may serve to charge battery 806.

Connector panel 808 may serve to connect different devices and components to surgical robot 4 and/or associated components and modules. Connector panel 808 may contain one or more ports that receive lines or connections from different components. For example, connector panel 808 may have a ground terminal port that may ground surgical robot 4 to other equipment, a port to connect foot pedal 880, a port to connect to tracking subsystem 830, which may include position sensor 832, camera converter 834, and DRA tracking cameras 870. Connector panel 808 may also include other ports to allow USB, Ethernet, HDMI communications to other components, such as computer 822. In accordance with some embodiments, the connector panel 808 can include a wired and/or wireless interface for operatively connecting one or more XR headsets 920 to the tracking subsystem 830 and/or the computer subsystem 820.

Control panel 816 may provide various buttons or indicators that control operation of surgical robot 4 and/or provide information from surgical robot 4 for observation by an operator. For example, control panel 816 may include buttons to power on or off surgical robot 4, lift or lower vertical column 16, and lift or lower stabilizers 855-858 that may be designed to engage casters 12 to lock surgical robot 4 from physically moving. Other buttons may stop surgical robot 4 in the event of an emergency, which may remove all motor power and apply mechanical brakes to stop all motion from occurring. Control panel 816 may also have indicators notifying the operator of certain system conditions such as a line power indicator or status of charge for battery 806. In accordance with some embodiments, one or more XR headsets 920 may communicate, e.g. via the connector panel 808, to control operation of the surgical robot 4 and/or to received and display information generated by surgical robot 4 for observation by persons wearing the XR headsets 920.

Computer 822 of computer subsystem 820 includes an operating system and software to operate assigned functions of surgical robot 4. Computer 822 may receive and process information from other components (for example, tracking subsystem 830, platform subsystem 802, and/or motion control subsystem 840) in order to display information to the operator. Further, computer subsystem 820 may provide output through the speaker 826 for the operator. The speaker may be part of the surgical robot, part of an XR headset 920, or within another component of the surgical system 2. The display 824 may correspond to the display 34 shown in Figures 1 and 2.

Tracking subsystem 830 may include position sensor 832 and camera converter 834. Tracking subsystem 830 may correspond to the camera tracking system component 6 of Figure 3. The DRA tracking cameras 870 operate with the position sensor 832 to determine the pose of DRAs 52. This tracking may be conducted in a manner consistent with the present disclosure including the use of infrared or visible light technology that tracks the location of active or passive elements of DRAs 52, such as LEDs or reflective markers, respectively.

Functional operations of the tracking subsystem 830 and the computer subsystem 820 can be included in the computer platform 910, which can be transported by the camera tracking system component 6' of Figures 3A and 3B. The tracking subsystem 830 can be configured to determine the poses, e.g., location and angular orientation of the tracked DRAs. The computer platform 910 can also include a navigation controller that is configured to use the determined poses to provide navigation information to users that guides their movement of tracked tools relative to position-registered patient images and/or tracked anatomical structures during a planned surgical procedure. The computer platform 910 can display information on the display of Figures 3B and 3C and/or to one or more XR headsets 920. The computer platform 910, when used with a surgical robot, can be configured to communicate with the computer subsystem 820 and other subsystems of Figure 8 to control movement of the end effector 26. For example, as will be explained below the computer platform 910 can generate a graphical representation of a patient's anatomical structure, surgical tool, user's hand, etc. with a displayed size, shape, color, and/or pose that is controlled based on the determined pose(s) of one or more the tracked DRAs, and which the graphical representation that is displayed can be dynamically modified to track changes in the determined poses over time.

Motion control subsystem 840 may be configured to physically move vertical column 16, upper arm 18, lower arm 20, or rotate end effector coupler 22. The physical movement may be conducted through the use of one or more motors 850-854. For example, motor 850 may be configured to vertically lift or lower vertical column 16. Motor 851 may be configured to laterally move upper arm 18 around a point of engagement with vertical column 16 as shown in Figure 2. Motor 852 may be configured to laterally move lower arm 20 around a point of engagement with upper arm 18 as shown in Figure 2. Motors 853 and 854 may be configured to move end effector coupler 22 to provide translational movement and rotation along in about three-dimensional axes. The computer platform 910 shown in Figure 9 can provide control input to the controller 846 that guides movement of the end effector coupler 22 to position a passive end effector, which is connected thereto, with a planned pose (i.e., location and angular orientation relative to defined 3D orthogonal reference axes) relative to an anatomical structure that is to be operated on during a planned surgical procedure. Motion control subsystem 840 may be configured to measure position of the end effector coupler 22 and/or the end effector 26 using integrated position sensors (e.g. encoders).

Figure 9 illustrates a block diagram of components of a surgical system that includes imaging devices (e.g., C-Arm 104, O-Arm 106, etc.) connected to a computer platform 910 which can be operationally connected to a camera tracking system component 6 (Fig. 3A) or 6' (Figs. 3B,3C) and/or to surgical robot 4 according to some embodiments of the present disclosure. Alternatively, at least some operations disclosed herein as being performed by the computer platform 910 may additionally or alternatively be performed by components of a surgical system.

Referring to Figure 9, the computer platform 910 includes a display 912, at least one processor circuit 914 (also referred to as a processor for brevity), at least one memory circuit 916 (also referred to as a memory for brevity) containing computer readable program code 918, and at least one network interface 902 (also referred to as a network interface for brevity). The display 912 may be part of an XR headset 920 in accordance with some embodiments of the present disclosure. The network interface 902 can be configured to connect to a C-Arm imaging device 104 in Figure 10, an O-Arm imaging device 106 in Figure 11, another medical imaging device, an image database 950 containing patient medical images, components of the surgical robot 4, and/or other electronic equipment.

When used with a surgical robot 4, the display 912 may correspond to the display 34 of Figure 2 and/or the tablet 890 of Figure 8 and/or the XR headset 920 that is operatively connected to the surgical robot 4, the network interface 902 may correspond to the platform network interface 812 of Figure 8, and the processor 914 may correspond to the computer 822 of Figure 8. The network interface 902 of the XR headset 920 may be configured to communicate through a wired network, e.g., thin wire ethernet, and/or through wireless RF transceiver link according to one or more wireless communication protocols, e.g., WLAN, 3GPP 4G and/or 5G (New Radio) cellular communication standards, etc.

The processor 914 may include one or more data processing circuits, such as a general purpose and/or special purpose processor, e.g., microprocessor and/or digital signal processor. The processor 914 is configured to execute the computer readable program code 918 in the memory 916 to perform operations, which may include some or all of the operations described herein as being performed for surgery planning, navigated surgery, and/or robotic surgery.

The computer platform 910 can be configured to provide surgery planning functionality. The processor 914 can operate to display on the display device 912 and/or on the XR headset 920 an image of an anatomical structure, e.g., vertebra, that is received from one of the imaging devices 104 and 106 and/or from the image database 950 through the network interface 920. The processor 914 receives an operator's definition of where the anatomical structure shown in one or more images is to have a surgical procedure, e.g., screw placement, such as by the operator touch selecting locations on the display 912 for planned procedures or using a mousebased cursor to define locations for planned procedures. When the image is displayed in the XR headset 920, the XR headset can be configured to sense in gesture-based commands formed by the wearer and/or sense voice based commands spoken by the wearer, which can be used to control selection among menu items and/or control how objects are displayed on the XR headset 920 as will be explained in further detail below.

The computer platform 910 can be configured to enable anatomy measurement, which can be particularly useful for knee surgery, like measurement of various angles determining center of hip, center of angles, natural landmarks (e.g. transepicondylar line, Whitesides line, posterior condylar line), etc. Some measurements can be automatic while some others can involve human input or assistance. The computer platform 910 may be configured to allow an operator to input a choice of the correct implant for a patient, including choice of size and alignment. The computer platform 910 may be configured to perform automatic or semiautomatic (involving human input) segmentation (image processing) for CT images or other medical images. The surgical plan for a patient may be stored in a cloud-based server, which may correspond to database 950, for retrieval by the surgical robot 4.

During orthopedic surgery, for example, a surgeon may choose which cut to make (e.g. posterior femur, proximal tibia etc.) using a computer screen (e.g. touchscreen) or extended reality ("XR") interaction (e.g., hand gesture based commands and/or voice based commands) via, e.g., the XR headset 920. The computer platform 910 can generate navigation information which provides visual guidance to the surgeon for performing the surgical procedure. When used with the surgical robot 4, the computer platform 910 can provide guidance that allows the surgical robot 4 to automatically move the end effector 26 to a target pose so that the surgical tool is aligned with a target location to perform the surgical procedure on an anatomical structure.

In some embodiments, the surgical system 900 can use two DRAs to track patient anatomy position, such as one connected to patient tibia and one connected to patient femur. The system 900 may use standard navigated instruments for the registration and checks (e.g. a pointer similar to the one used in Globus ExcelsiusGPS system for spine surgery).

A particularly challenging task in navigated surgery is how to plan the position of an implant in spine, knee, and other anatomical structures where surgeons struggle to perform the task on a computer screen which is a 2D representation of the 3D anatomical structure. The system 900 could address this problem by using the XR headset 920 to display a three-dimensional ("3D") computer generated representations of the anatomical structure and a candidate implant device. The computer generated representations are scaled and posed relative to each other on the display screen under guidance of the computer platform 910 and which can be manipulated by a surgeon while viewed through the XR headset 920. A surgeon may, for example, manipulate the displayed computer-generated representations of the anatomical structure, the implant, a surgical tool, etc., using hand gesture based commands and/or voice based commands that are sensed by the XR headset 920.

For example, a surgeon can view a displayed virtual handle on a virtual implant, and can manipulate (e.g., grab and move) the virtual handle to move the virtual implant to a desired pose and adjust a planned implant placement relative to a graphical representation of an anatomical structure. Afterward, during surgery, the computer platform 910 could display navigation information through the XR headset 920 that facilitates the surgeon's ability to more accurately follow the surgical plan to insert the implant and/or to perform another surgical procedure on the anatomical structure. When the surgical procedure involves bone removal, the progress of bone removal, e.g., depth of cut, can be displayed in real-time through the XR headset 920. Other features that may be displayed through the XR headset 920 can include, without limitation, gap or ligament balance along a range of joint motion, contact line on the implant along the range of joint motion, ligament tension and/or laxity through color or other graphical renderings, etc.

The computer platform 910, in some embodiments, can allow planning for use of standard surgical tools and/or implants, e.g., posterior stabilized implants and cruciate retaining implants, cemented and cementless implants, revision systems for surgeries related to, for example, total or partial knee and/or hip replacement and/or trauma.

An automated imaging system can be used in conjunction with the computer platform 910 to acquire pre-operative, intra-operative, post-operative, and/or real-time image data of an anatomical structure. Example automated imaging systems are illustrated in Figures 10 and 11. In some embodiments, the automated imaging system is a C-arm 104 (Fig. 10) imaging device or an O-arm^{®} 106 (Fig. 11). (O-arm^{®} is copyrighted by Medtronic Navigation, Inc. having a place of business in Louisville, Colo., USA). It may be desirable to take x-rays of a patient from a number of different positions, without the need for frequent manual repositioning of the patient which may be required in an x-ray system. C-arm 104 x-ray diagnostic equipment may solve the problems of frequent manual repositioning and may be well known in the medical art of surgical and other interventional procedures. As illustrated in Figure 10, a C-arm includes an elongated C-shaped member terminating in opposing distal ends 112 of the "C" shape. C-shaped member is attached to an x-ray source 114 and an image receptor 116. The space within C-arm 104 of the arm provides room for the physician to attend to the patient substantially free of interference from the x-ray support structure.

The C-arm is mounted to enable rotational movement of the arm in two degrees of freedom, (i.e. about two perpendicular axes in a spherical motion). C-arm is slidably mounted to an x-ray support structure, which allows orbiting rotational movement of the C-arm about its center of curvature, which may permit selective orientation of x-ray source 114 and image receptor 116 vertically and/or horizontally. The C-arm may also be laterally rotatable, (i.e. in a perpendicular direction relative to the orbiting direction to enable selectively adjustable positioning of x-ray source 114 and image receptor 116 relative to both the width and length of the patient). Spherically rotational aspects of the C-arm apparatus allow physicians to take x-rays of the patient at an optimal angle as determined with respect to the particular anatomical condition being imaged.

The O-arm^{®} 106 illustrated in Figure 11 includes a gantry housing 124 which may enclose an image capturing portion, not illustrated. The image capturing portion includes an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of the patient to be acquired from multiple directions or in multiple planes.

The O-arm^{®} 106 with the gantry housing 124 has a central opening for positioning around an object to be imaged, a source of radiation that is rotatable around the interior of gantry housing 124, which may be adapted to project radiation from a plurality of different projection angles. A detector system is adapted to detect the radiation at each projection angle to acquire object images from multiple projection planes in a quasi-simultaneous manner. The gantry may be attached to a support structure O-arm^{®} support structure, such as a wheeled mobile cart with wheels, in a cantilevered fashion. A positioning unit translates and/or tilts the gantry to a planned position and orientation, preferably under control of a computerized motion control system. The gantry may include a source and detector disposed opposite one another on the gantry. The source and detector may be secured to a motorized rotor, which may rotate the source and detector around the interior of the gantry in coordination with one another. The source may be pulsed at multiple positions and orientations over a partial and/or full three hundred and sixty degree rotation for multi-planar imaging of a targeted object located inside the gantry. The gantry may further comprise a rail and bearing system for guiding the rotor as it rotates, which may carry the source and detector. Both and/or either O-arm^{®} 106 and C-arm 104 may be used as automated imaging system to scan a patient and send information to the surgical system 2.

Images captured by an imaging system can be displayed on the XR headset 920 and/or another display device of the computer platform 910, the surgical robot 4, and/or another component of the surgical system 900. The XR headset 920 may be connected to one or more of the imaging devices 104 and/or 106 and/or to the image database 950, e.g., via the computer platform 910, to display images therefrom. A user may provide control inputs through the XR headset 920, e.g., gesture and/or voice based commands, to control operation of one or more of the imaging devices 104 and/or 106 and/or the image database 950.

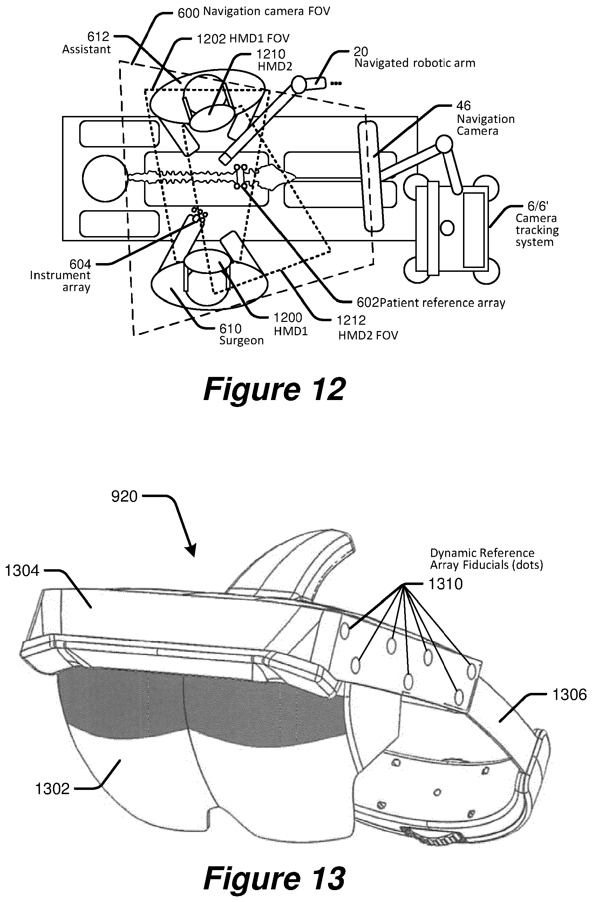

Figure 12 illustrates a block diagram view of the components of a surgical system that include a pair of XR headsets 1200 and 1210 (head-mounted displays HMD1 and HMD2), which may correspond to the XR headset 920 shown in Figure 13 and operate in accordance with some embodiments of the present disclosure.

Referring to the example scenario of Figure 12, the assistant 612 and surgeon 610 are both wearing the XR headsets 1210 and 1210, respectively. It is optional for the assistant 612 to wear the XR headset 1210. The XR headsets 1200 and 1210 are configured to provide an interactive environment through which the wearers can view and interact with information related to a surgical procedure as will be described further below. This interactive XR based environment may eliminate a need for the tech personnel 614 to be present in the operating room and may eliminate a need for use of the display 34 shown in Figure 6. Each XR headset 1200 and 1210 can include one or more cameras that are be configured to provide an additional source of tracking of DRAs or other reference arrays attached to instruments, an anatomical structure, the end effector 26, and/or other equipment. In the example of Figure 12, XR headset 1200 has a field-of-view ("FOV") 1202 for tracking DRAs and other objects, XR headset 1210 has a FOV 1212 partially overlapping FOV 1202 for tracking DRAs and other objects, and the navigation cameras 46 has another FOV 600 partially overlapping FOVs 1202 and 1212 for tracking DRAs and other objects.

If one or more cameras is obstructed from viewing a DRA attached to a tracked object, e.g., a surgical instrument, but the DRA is in view of one or more other cameras the tracking subsystem 830 and/or navigation controller 828 can continue to track the object seamlessly without loss of navigation. Additionally, if there is partial occlusion of the DRA from the perspective of one camera, but the entire DRA is visible via multiple camera sources, the tracking inputs of the cameras can be merged to continue navigation of the DRA. One of the XR headsets and/or the navigation cameras 46 may view and track the DRA on another one of the XR headsets to enable the computer platform 910 (Figs. 9 and 14), the tracking subsystem 830, and/or another computing component to determine the pose of the DRA relative to one or more defined coordinate systems, e.g., of the XR headsets 1200/1210, the navigation cameras 46, and/or another coordinate system defined for the patient, table, and/or room.

The XR headsets 1200 and 1210 can be operatively connected to view video, pictures, and/or other information received from and/or to provide commands that control various equipment in the surgical room, including but not limited to neuromonitoring, microscopes, video cameras, and anesthesia systems. Data from the various equipment may be processed and displayed within the headset, for example the display of patient vitals or the microscope feed.

### Example XR Headset Components and Integration to Navigated Surgery, Surgical Robots, and Other Equipment

Figure 13 illustrates an XR headset 920 which is configured in accordance with some embodiments of the present disclosure. The XR headset includes a headband 1306 configured to secure the XR headset to a wearer's head, an electronic component enclosure 1304 supported by the headband 1306, and a display screen 1302 that extends laterally across and downward from the electronic component enclosure 1304. The display screen 1302 may be a see-through LCD display device or a semi-reflective lens that reflects images projected by a display device toward the wearer's eyes. A set of DRA fiducials, e.g., dots are painted or attached in a spaced apart known arranged on one or both sides of the headset. The DRA on the headset enables the navigation cameras on the auxiliary tracking bar to track pose of the headset 920 and/or enables another XR headset to track pose of the headset 920.

The display screen 1302 operates as a see-through display screen, also referred to as a combiner, that reflects light from display panels of a display device toward the user's eyes. The display panels can be located between the electronic component enclosure and the user's head, and angled to project virtual content toward the display screen 1302 for reflection toward the user's eyes. The display screen 1302 is semi-transparent and semi-reflective allowing the user to see reflected virtual content superimposed on the user's view of a real-world scene. The display screen 1302 may have different opacity regions, such as the illustrated upper laterally band which has a higher opacity than the lower laterally band. Opacity of the display screen 1302 may be electronically controlled to regulate how much light from the real-world scene passes through to the user's eyes. A high opacity configuration of the display screen 1302 results in high-contrast virtual images overlaid on a dim view of the real-world scene. A low opacity configuration of the display screen 1302 can result in more faint virtual images overlaid on a clearer view of the real-world scene. The opacity may be controlled by applying an opaque material on a surface of the display screen 1302.

According to some embodiments the surgical system includes an XR headset 920 and an XR headset controller, e.g., controller 1430 in Fig. 14 or controller 3410 in Fig. 34. The XR headset 920 is configured to be worn by a user during a surgical procedure and has a see-through display screen 1302 that is configured to display an XR image and to allow at least a portion of a real-world scene to pass therethrough for viewing by the user. The XR headset 920 also includes an opacity filter positioned between at least one of the user's eyes and the real-world scene when the see-through display screen 1302 is viewed by the user. The opacity filter is configured to provide opaqueness to light from the real-world scene. The XR headset controller is configured to communicate with a navigation controller, e.g., controller(s) 828A, 828B, and/or 828C in Fig. 14, to receive navigation information from the navigation controller which provides guidance to the user during the surgical procedure on an anatomical structure, and is further configured to generate the XR image based on the navigation information for display on the see-through display screen 1302.

Opacity of the display screen 1302 may be configured as a gradient having a more continuously changing opacity with distance downward from a top portion of the display screen 1302. The gradient's darkest point can be located at the top portion of the display screen 1302, and gradually becoming less opaque further down on the display screen 1302 until the opacity is transparent or not present. In an example further embodiment, the gradient can change from about 90% opacity to entirely transparent approximately at the mid-eye level of the display screen 1302. With the headset properly calibrated and positioned, the mid-eye level can correspond to the point where the user would look straight out, and the end of the gradient would be located at the "horizon" line of the eye. The darker portion of the gradient will allow crisp, clear visuals of the virtual content and help to block the intrusive brightness of the overhead operating room lights.

Using an opacity filter in this manner enables the XR headset 920 to provide virtual reality ("VR") capabilities, by substantially or entirely blocking light from the real-world scene, along an upper portion of the display screen 1302 and to provide AR capabilities along a middle or lower portion of the display screen 1302. This allows the user to have the semi-translucence of AR where needed and allowing clear optics of the patient anatomy during procedures. Configuring the display screen 1302 as a gradient instead of as a more constant opacity band can enable the wearer to experience a more natural transition between a more VR type view to a more AR type view without experiencing abrupt changes in brightness of the real-world scene and depth of view that may otherwise strain the eyes such as during more rapid shifting between upward and downward views.

The display panels and display screen 1302 can be configured to provide a wide field of view see-through XR display system. In one example configuration they provide an 80° diagonal field-of-view ("FOV") with 55° of vertical coverage for a user to view virtual content. Other diagonal FOV angles and vertical coverage angles can be provided through different size display panels, different curvature lens, and/or different distances and angular orientations between the display panels and curved display screen 1302. In some embodiments, having an XR headset with a wide field of view allows the XR headset to display real-world registered XR images to a user while the user's field of vision is not centered on the real-world coordinates associated with the real-world registered XR image.

Figure 14 illustrates electrical components of the XR headset 920 that can be operatively connected to the computer platform 910, to one or more of the imaging devices, such as the C-arm imaging device 104, the O-arm imaging device 106, and/or the image database 950, and/or to the surgical robot 800 in accordance with various embodiments of the present disclosure.

The XR headset 920 provides an improved human interface for performing navigated surgical procedures. The XR headset 920 can be configured to provide functionalities, e.g., via the computer platform 910, that include without limitation any one or more of: identification of hand gesture based commands and/or voice based commands, display XR graphical objects on a display device 1450. The display device 1450 may a video projector, flat panel display, etc., which projects the displayed XR graphical objects on the display screen 1302. The user can view the XR graphical objects as an overlay anchored to particular real-world objects viewed through the display screen 1302 (Fig. 13). The XR headset 920 may additionally or alternatively be configured to display on the display screen 1450 video feeds from cameras mounted to one or more XR headsets 920 and other cameras.

Electrical components of the XR headset 920 can include a plurality of cameras 1440, a microphone 1442, a gesture sensor 1444, a pose sensor (e.g., inertial measurement unit ("IMU")) 1446, a display module 1448 containing the display device 1450, and a wireless/wired communication interface 1452. As will be explained below, the cameras 1440 of the XR headset may be visible light capturing cameras, near infrared capturing cameras, or a combination of both.

The cameras 1440 may be configured operate as the gesture sensor 1444 by capturing for identification user hand gestures performed within the field of view of the camera(s) 1440. Alternatively the gesture sensor 1444 may be a proximity sensor and/or a touch sensor that senses hand gestures performed proximately to the gesture sensor 1444 and/or senses physical contact, e.g. tapping on the sensor or the enclosure 1304. The pose sensor 1446, e.g., IMU, may include a multi-axis accelerometer, a tilt sensor, and/or another sensor that can sense rotation and/or acceleration of the XR headset 920 along one or more defined coordinate axes. Some or all of these electrical components may be contained in the component enclosure 1304 or may be contained in another enclosure configured to be worn elsewhere, such as on the hip or shoulder.

As explained above, the surgical system 2 includes a camera tracking system component 6/6' and a tracking subsystem 830 which may be part of the computer platform 910. The surgical system may include imaging devices (e.g., C-arm 104, O-arm 106, and/or image database 950) and/or a surgical robot 4. The tracking subsystem 830 is configured to determine a pose of DRAs attached to an anatomical structure, an end effector, a surgical tool, etc. A navigation controller 828 is configured to determine a target pose for the surgical tool relative to an anatomical structure based on a surgical plan, e.g., from a surgical planning function performed by the computer platform 910 of Fig. 9, defining where a surgical procedure is to be performed using the surgical tool on the anatomical structure and based on a pose of the anatomical structure determined by the tracking subsystem 830. The navigation controller 828 may be further configured to generate steering information based on the target pose for the surgical tool, the pose of the anatomical structure, and the pose of the surgical tool and/or the end effector, where the steering information indicates where the surgical tool and/or the end effector of a surgical robot should be moved to perform the surgical plan.

The electrical components of the XR headset 920 can be operatively connected to the electrical components of the computer platform 910 through a wired/wireless interface 1452. The electrical components of the XR headset 920 may be operatively connected, e.g., through the computer platform 910 or directly connected, to various imaging devices, e.g., the C-arm imaging device 104, the I/O-arm imaging device 106, the image database 950, and/or to other medical equipment through the wired/wireless interface 1452.

The surgical system 2 further includes at least one XR headset controller 1430 (also referred to as "XR headset controller" for brevity) that may reside in the XR headset 920, the computer platform 910, and/or in another system component connected via wired cables and/or wireless communication links. Various functionality is provided by software executed by the XR headset controller 1430. The XR headset controller 1430 is configured to receive navigation information from the navigation controller 828 which provides guidance to the user during the surgical procedure on an anatomical structure, and is configured to generate an XR image based on the navigation information for display on the display device 1450 for projection on the see-through display screen 1302.

The configuration of the display device 1450 relative to the display screen (also referred to as "see-through display screen ") 1302 is configured to display XR images in a manner such that when the user wearing the XR headset 920 looks through the display screen 1302 the XR images appear to be in the real world. The display screen 1302 can be positioned by the headband 1306 in front of the user's eyes.

The XR headset controller 1430 can be within a housing that is configured to be worn on a user's head or elsewhere on the user's body while viewing the display screen 1302 or may be remotely located from the user viewing the display screen 1302 while being communicatively connected to the display screen 1302. The XR headset controller 1430 can be configured to operationally process signaling from the cameras 1440, the microphone 142, and/or the pose sensor 1446, and is connected to display XR images on the display device 1450 for user viewing on the display screen 1302. Thus, the XR headset controller 1430 illustrated as a circuit block within the XR headset 920 is to be understood as being operationally connected to other illustrated components of the XR headset 920 but not necessarily residing within a common housing (e.g., the electronic component enclosure 1304 of Fig. 13) or being otherwise transportable by the user. For example, the XR headset controller 1430 may reside within the computer platform 910 which, in turn, may reside within a housing of the computer tracking system component 6' shown in Figures 3B and 3C.

### Example User Views through the XR Headset

The XR headset operations can display both 2D images and 3D models on the display screen 1302. The 2D images may preferably be displayed in a more opaque band of the display screen 1302 (upper band) and the 3D model may be more preferably displayed in the more transparent band of the display screen 1302, otherwise known as the environmental region (bottom band). Below the lower band where the display screen 1302 ends the wearer has an unobstructed view of the surgical room. It is noted that where XR content is display on the display screen 1302 may be fluidic. It is possible that where the 3D content is displayed moves to the opaque band depending on the position of the headset relative to the content, and where 2D content is displayed can be placed in the transparent band and stabilized to the real world. Additionally, the entire display screen 1302 may be darkened under electronic control to convert the headset into virtual reality for surgical planning or completely transparent during the medical procedure. As explained above, the XR headset 920 and associated operations not only support navigated procedures, but also can be performed in conjunction with robotically assisted procedures.

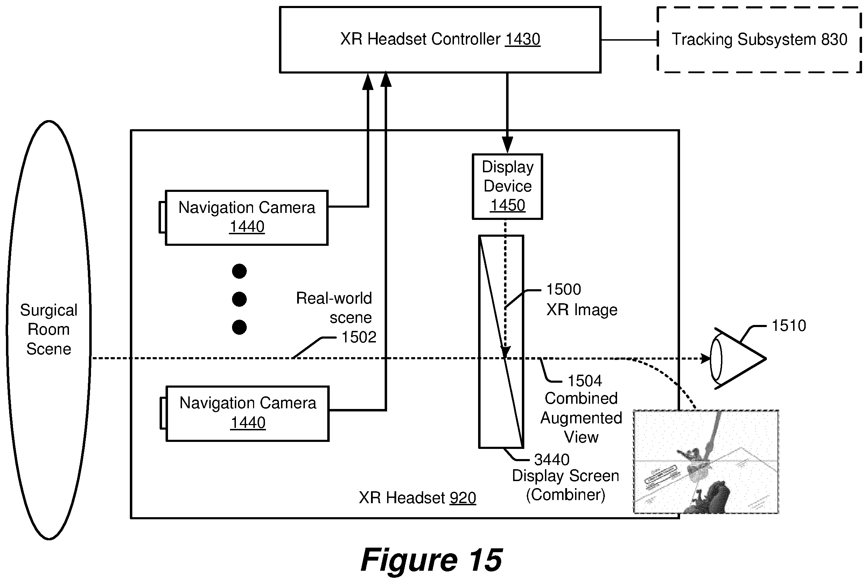

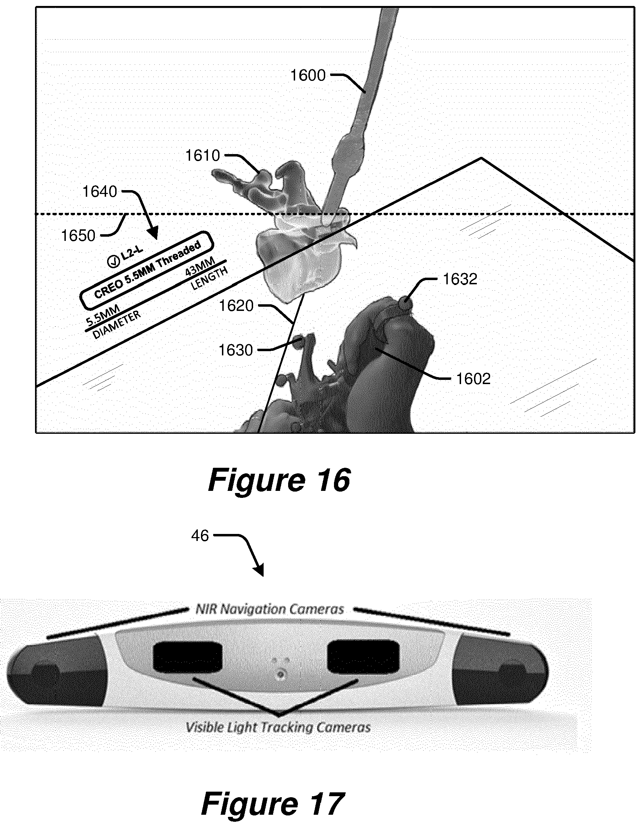

Figure 16 illustrates an example view through the display screen 1302 of the XR headset 920 for providing navigation assistance to a user who is manipulating a surgical tool 1602 during a medical procedure in accordance with some embodiments of the present disclosure. Referring to Figure 16, when the surgical tool 1602 is brought in vicinity of a tracked anatomical structure so that dynamic reference arrays 1630 and 1632, connected to the surgical tool 1602, become within the field of view of the cameras 1440 (Fig. 15) and/or 46 (Fig. 6), a graphical representation 1600 of the tool can be displayed in 2D and/or 3D images in relation to a graphical representation 1610 of the anatomical structure. The user can use the viewed graphical representations to adjust a trajectory 1620 of the surgical tool 1602, which can be illustrated as extending from the graphical representation 2000 of the tool through the graphical representation 1610 of the anatomical structure. The XR headset 920 may also display textual information and other objects 1640. The dashed line 1650 extending across the viewed display screen represents an example division between different opacity level upper and lower bands.

Other types of XR images (virtual content) that can be displayed on the display screen 1302 can include, but are not limited to any one or more of:
I) 2D Axial, Sagittal and/or Coronal views of patient anatomy;
2) overlay of planned vs currently tracked tool and surgical implant locations;
3) gallery of preoperative images;
4) video feeds from microscopes and other similar systems or remote video conferencing;
5) options and configuration settings and buttons;
6) floating 3D models of patient anatomy with surgical planning information;
7) real-time tracking of surgical instruments relative to floating patient anatomy;
8) augmented overlay of patient anatomy with instructions and guidance; and
9) augmented overlay of surgical equipment.

### Example Configuration of Cameras for Tracking System Component

Figure 17 illustrates example configuration of an auxiliary tracking bar 46 having two pairs of stereo navigation cameras configured in accordance with some embodiments of the present disclosure. The auxiliary tracking bar 46 is part of the camera tracking system component of Figures 3A, 3B, and 3C. The stereo navigation cameras include a stereo pair of spaced apart visible light capturing cameras and another stereo pair of spaced apart near infrared capturing cameras, in accordance with one embodiment. Alternatively, only one stereo pair of visible light capturing cameras or only one stereo pair of near infrared capture cameras can used in the auxiliary tracking bar 46. Any plural number of near infrared and/or visible light cameras can be used.

### Pose Measurement Chaining

As explained above, navigated surgery can include computer vision tracking and determination of pose (e.g., position and orientation in a six degree-of-freedom coordinate system) of surgical instruments, such as by determining pose of attached DRAs that include spaced apart fiducials, e.g., disks or spheres, arranged in a known manner. The computer vision uses spaced apart navigation cameras, e.g., stereo cameras, that are configured to capture near infrared and/or visible light. In this scenario, there are three parameters jointly competing for optimization: (1) accuracy, (2) robustness, and (3) user ergonomics during a surgical procedure.

Some further aspects of the present disclosure are directed to computer operations that combine (chain) measured poses in ways that can improve optimization of one or more of the above three parameters by incorporating additional navigation cameras mounted to one or more XR headsets. As shown in Figure 17, a stereo pair of visible light tracking cameras and another stereo pair of near infrared tracking navigation cameras can be attached to the auxiliary tracking bar of the camera tracking system component in accordance with some embodiments of the present disclosure. Operational algorithms are disclosed that analyze the pose of DRAs that are fully observed or partially observed (e.g., when less than all of the fiducials of a DRA are viewed by a pair of stereo cameras), and combine the observed poses or partial poses in ways that can improve accuracy, robustness, and/or ergonomics during navigated surgery.

As explained above, the XR headset may be configured to augment a real-world scene with computer generated XR images. The XR headset may be configured to provide an XR viewing environment by displaying the computer generated XR images on a see-through display screen that allows light from the real-world scene to pass therethrough for combined viewing by the user. Alternatively, the XR headset may be configured to provide a VR viewing environment by preventing or substantially preventing light from the real-world scene from being directly viewed by the user along the viewing path of the displayed XR images. An XR headset can be configured to provide both AR and VR viewing environments. In one embodiment, both AR and VR viewing environments are provided by lateral bands of substantially differing opacity arranged between the see-through display screen and the real-world scene, so that a VR viewing environment is provided for XR images aligned with a high opacity band and an AR viewing environment is provided for XR images aligned with the low opacity band. In another embodiment, both AR and VR viewing environments are provided by computer adjustable control of an opacity filter that variably constrains how much light from the real-world scene passes through a see-through display screen for combining with the XR images viewed by the user. Thus, the XR headset can also be referred to as an AR headset or a VR headset.

As was also explained above, the XR headset can include near infrared tracking cameras and/or visible light tracking cameras that are configured to track fiducials of DRAs connected to surgical instruments, patient anatomy, other XR headset(s), and/or a robotic end effector. Using near infrared tracking and/or visible light tracking on the XR headset provides additional tracking volume coverage beyond what cameras on a single auxiliary tracking bar can provide. Adding near infrared tracking cameras to the existing auxiliary tracking bar allows for the headset location to be tracked more robustly but less accurately than in visible light. Mechanically calibrating the visible and near infrared tracking coordinate systems enables the coordinate systems to be aligned sufficiently to perform 3D DRA fiducials triangulation operations using stereo matching to jointly identify pose of the DRA fiducials between the visible and near infrared tracking coordinate systems. Using both visible and near infrared tracking coordinate systems can enable any one or more of: (a) identifying tools that would not be identified using a single coordinate system; (b) increased pose tracking accuracy; (c) enabling a wider range of motion without losing tracking of surgical instruments, patient anatomy, and/or a robotic end effector; and (d) naturally track an XR headset in the same coordinate system as the navigated surgical instruments.

### Virtual Model of Planned Instrument Attachment to Reduce Incorrect Selection

During a surgical operation, an instrument attachment can be attached to an instrument. The instrument attachment can be an implant (e.g., a pedicle screw or an expandable cage) or a tool enhancer (e.g., a curved tip). In some examples, a surgeon selects the instrument attachment and attaches the instrument attachment to the instrument. In additional or alternative examples, the surgeon requests the instrument attachment and an assistant (e.g., a technician or nurse) provides the instrument attachment and/or an instrument with the instrument attachment attached to the surgeon or a surgical robot. In additional or alternative examples, a surgical plan instructs a surgeon, assistant, or surgical robot to use the instrument attachment and the corresponding surgeon, assistant, or surgical robot selects the instrument attachment. During this operation (of requesting/selecting the instrument attachment), miscommunication can result in an incorrect instrument attachment being provided. For example, an implant of the wrong size (e.g., length, width, or diameter) can be provided. There are numerous different options for implant in terms of size and product family so a nurse or technician could potentially select and ready the wrong implant. Moreover, in some examples, the surgeon or surgical robot may be unaware that the provided instrument attachment is incorrect.

An incorrect instrument attachment that is detected prior to insertion of the instrument attachment in a patient, but after the sterility of the instrument attachment has been compromised, results in an wasted (e.g., unusable) instrument attachment. More serious complications can arise if at attempt at insertion or use of the instrument attachment is made. For example, an implant that is the wrong size can cause damage to a patient during attempted implantation in the patient.

Various embodiments herein describe a virtual model of a planned instrument attachment being displayed to a surgeon by an XR headset to allow the surgeon to verify a physical (e.g., real-world) instrument attachment that has been provided is the planned instrument attachment. Displaying a virtual model of the planned instrument attachment decreases the likelihood that the wrong instrument attachment is used (e.g., the wrong implant is put into a patient).

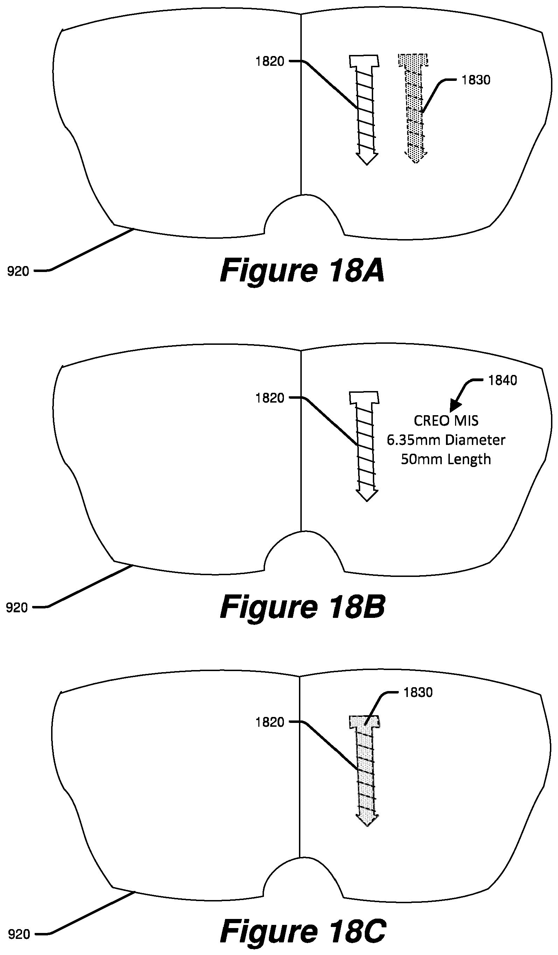

Figures 18A-C illustrate examples of a view through XR headset 920. In Figure 18A, the virtual model 1830 of a planned instrument attachment is displayed at a predetermined offset and/or pose relative to the XR headset 920. The planned instrument can be selected by the surgeon via the XR headset 920 or based on a predetermined surgical plan. In this example, a physical instrument attachment 1820 (e.g., a real-world instrument attachment) is also visible. The virtual model 1830 includes a three-dimensional representation of the planned instrument attachment. In Figure 18B, the virtual model 1830 includes virtual text describing features (e.g., size, thread type, shape, writings) of the planned instrument attachment. In some examples, a virtual model includes both a three-dimensional representation and virtual text describing features of the planned instrument attachment. A surgeon viewing the physical instrument attachment 1820 and the virtual model 1830 via the XR headset 920 can determine whether the planned physical instrument has been provided (e.g., whether the physical instrument 1820 matches the planned instrument).

In some embodiments, the virtual model 1830 of the planned instrument attachment is displayed based on a pose of the XR headset 920 relative to a pose of the physical instrument attachment 1820. In some examples, the XR headset determines a scale and/or orientation of the virtual model 1830 of the planned instrument attachment based on the pose of the pose of the XR headset 920 relative to the pose of the physical instrument attachment 1820. As a result, the virtual model 1830 of the planned instrument attachment is displayed to the user at the same scale and/or orientation as a physical version of the planned instrument attachment would appear if at the pose of the physical instrument attachment 1820.

In Figure 18C, the virtual model 1830 of the planned instrument attachment is displayed as an overlay of the physical instrument attachment 1820. In this example, the virtual model 1830 is partially transparent such that the physical instrument attachment 1820 is visible through the virtual model 1830 and the surgeon can observe if there are any differences between the virtual model 1830 and the physical instrument attachment 1820. In additional or alternative examples, the virtual model 1830 may be opaque (e.g., a solid color) and displayed as appearing directly in front of the physical instrument attachment 1820 such that if any portion of the physical instrument attachment 1820 is visible to the surgeon, the surgeon can confirm the physical instrument attachment 1820 is incorrect. In additional or alternative examples, the virtual model 1830 may be opaque and displayed as appearing directly behind the physical instrument attachment 1820 such that if any portion of the virtual model is visible, the surgeon can confirm the physical instrument attachment 1820 is incorrect. The surgeon may control (and switch between) the different poses of the virtual model 1830 relative to the physical instrument attachment 1820.

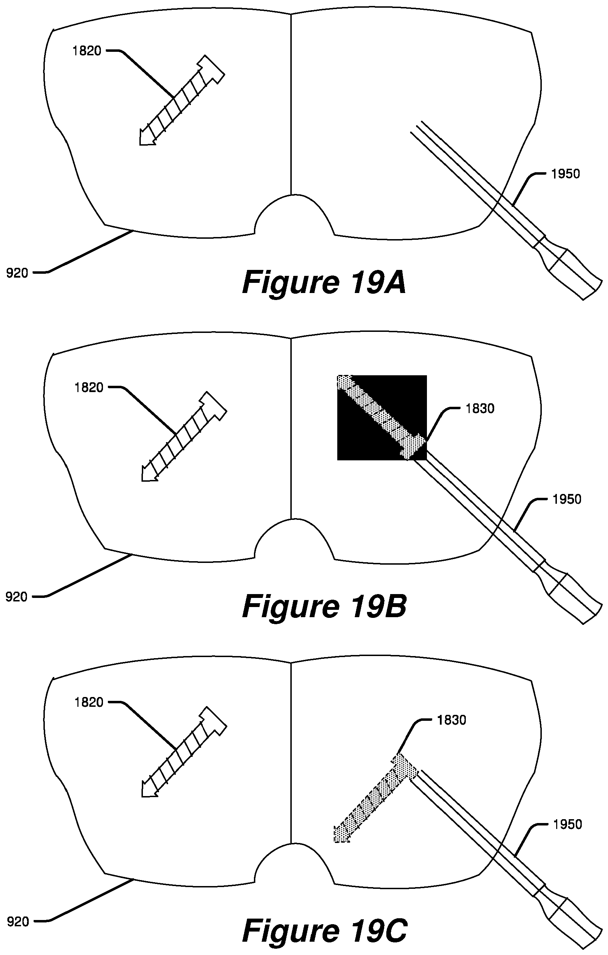

Figures 19A-C illustrate examples of a view through the XR headset 920 in which the physical instrument attachment 1820 is visible, but not attached to a physical (real-world) instrument 1950. In Figure 19A, the instrument 1950 is visible through a display of the XR headset 920. The surgeon may provide user input to the XR headset 920 or an XR headset controller to cause, as illustrated in Figure 19B, a virtual model 1830 of a planned instrument attachment to be displayed as attached to the instrument 1950. The instrument 1950 may include markers that are tracked by a camera tracking system, and the virtual model 1830 of the planned instrument attachment may be displayed based on a pose of the instrument 1950 relative to the XR headset 920 to cause the virtual model 1830 to appear as extending from an end of the instrument 1950 to which the planned instrument attachment would attach.

In some examples, the user input includes moving the instrument 1950 to a specific real-world position or orientation relative to the XR headset 920. In additional or alternative examples, the user input is during a planning stage and involves assigning the planned instrument attachment to the instrument 1950 such that the XR headset 920 automatically associates the planned instrument attachment with the instrument 1950.

In Figure 19C, the virtual model 1830 is displayed as attached to the instrument 1950, but at an orientation of the physical instrument attachment 1820. A camera tracking system can determine a pose of the physical instrument attachment 1820 and the XR headset can display the virtual model 1830 based on a pose of the physical instrument attachment 1820 and a pose of the instrument 1950 relative to a pose of the XR headset 920.

In some embodiments, the physical instrument attachment 1820 may be attached to the instrument 1950 and/or the surgeon may position the physical instrument attachment 1820 to be attached to the instrument 1950. If the virtual model 1830 and the physical instrument attachment 1820 lineup, the surgeon has an extra degree of certainty that the right selection has been made.

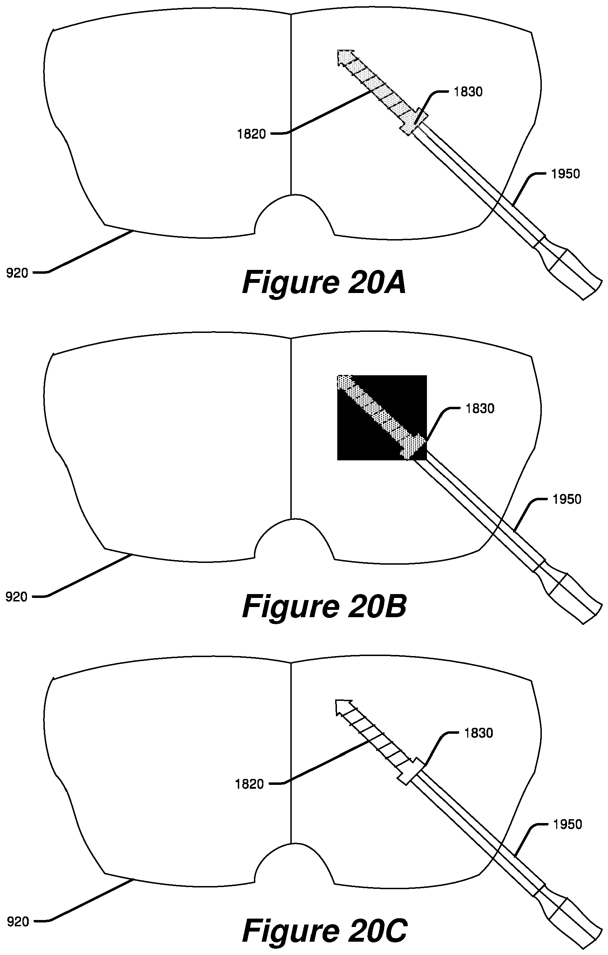

Figures 20A-C illustrate examples of a view through the XR headset 920 in which the virtual model 1830 is displayed as attached to an instrument 1950, which currently has the physical instrument attachment 1820 attached. In Figure 20A the virtual model 1830 is partially transparent and the physical instrument 1820 is partially visible through the virtual model 1830. The surgeon wearing the XR headset 920 can determine that the physical instrument attachment 1820 is correct based on comparing the size and features of the physical instrument attachment 1820 and the virtual model 1830.

In Figure 20B, the virtual model 1830 is opaque (e.g., a solid color) and displayed as appearing directly in front of the physical instrument attachment 1820. The surgeon wearing the XR headset 920 can determine that the physical instrument attachment 1820 is correct based on not being able to observe a portion of the physical instrument 1820 around the virtual model 1830.

In Figure 20C, the virtual model 1830 is opaque (e.g., a solid color) and displayed as appearing directly behind the physical instrument attachment 1820. The surgeon wearing the XR headset 920 can determine that the physical instrument attachment 1820 is correct based on not being able to observe a portion of the virtual model 1830 around the physical instrument attachment 1820.

In some embodiments, the surgeon can switch between the virtual model 1830 being displayed as partially transparent, opaque and in front of the physical instrument attachment 1820, and opaque and behind the physical instrument attachment 1820 in order to determine whether the physical instrument attachment 1820 matches the planned instrument attachment.

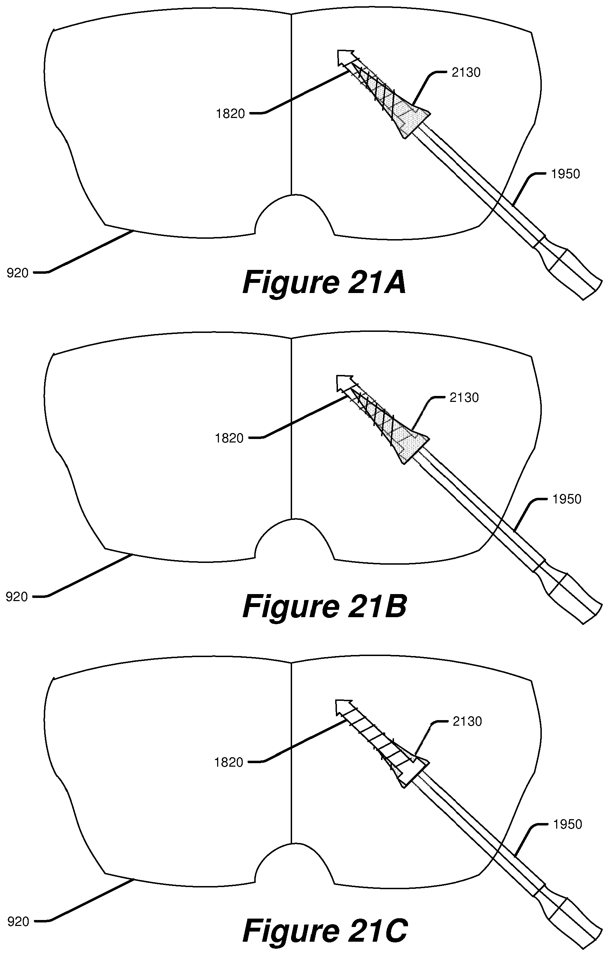

In the examples illustrated in Figures 18AC, 19A-C, and 20A-C the physical instrument attachment 1820 appears to match the virtual model 1830, therefore, a surgeon may determine that the physical instrument attachment 1820 is the correct and/or planned instrument attachment. In Figures 21A-C the physical instrument attachment 1820 appears to not match (e.g., has a different length, width, shape, and thread pattern) a virtual model 2130 of a planned instrument attachment.

Figures 21A-C illustrate examples of a view through the XR headset 920 in which the virtual model 2130 is displayed as attached to an instrument 1950, which currently has the physical instrument attachment 1820 attached. In Figure 21A the virtual model 2130 is partially transparent and the physical instrument 1820 is partially visible through the virtual model 2130. The surgeon wearing the XR headset 920 can determine that the physical instrument attachment 1820 is incorrect based on being able to observe differences in the physical instrument attachment 1820 and the virtual model 2130.

In Figure 21B, the virtual model 2130 is opaque (e.g., a solid color) and displayed as appearing directly in front of the physical instrument attachment 1820. The surgeon wearing the XR headset 920 can determine that the physical instrument attachment 1820 is incorrect based on being able to observe a portion of the physical instrument 1820 around the virtual model 2130.

In Figure 21C, the virtual model 2130 is opaque (e.g., a solid color) and displayed as appearing directly behind the physical instrument attachment 1820. The surgeon wearing the XR headset 920 can determine that the physical instrument attachment 1820 is incorrect based on being able to observe a portion of the virtual model 2130 around the physical instrument attachment 1820.

In some embodiments, the surgeon can switch between the virtual model 2130 being displayed as partially transparent, opaque and in front of the physical instrument attachment 1820, and opaque and behind the physical instrument attachment 1820 in order to determine whether the physical instrument attachment 1820 matches the planned instrument attachment.

In some embodiments, a surgical operation includes inserting an implant into a patient. An implant is a piece of hardware (e.g., a pedicle screw or an expandable cage) that will be inserted into a patient. The implant is selected by the surgeon based on a variety of different factors including the patient's age, bone health, vertebrae size, and the surgeon's preference. In additional or alternative embodiments, a surgical operation includes using an instrument with an instrument extension attached. An instrument extension is a piece of hardware (e.g., a curved tip) that is attached to an instrument to allow a surgeon to perform a specific surgical operation. The instrument extension is selected by the surgeon based on a variety of factors including a type of surgical operation, a location of the surgical operation, and surgeon's preference. Further description of a process for ensuring an instrument attachment is correct is provided below in regards to a surgical operation involving an implantation, but a similar process is applicable to a surgical operation involving an instrument extension.

Once a surgeon chooses which implant to use, the surgeon communicates this selection through navigation software or by telling the attending staff. Communication of the selection can be complicated, which can lead to miscommunication particularly given that an operating room is a high stress and often loud environment.

To help prevent this potentially disastrous situation, an XR headset can be used by the surgeon. The XR headset can first show a projection of the screw that the surgeon has selected as well as appropriate dimensions to confirm that the correct choice was made and communicated.

The surgeon would then confirm that the right implant was selected. Once the implant has been attached to an instrument and handed to the surgeon, the XR headset could then track the instrument and overlay a virtual image of the implant onto where it should be in reality. If the virtual image and the real-world implant appear to be mismatched in terms of size, shape, etc., then the surgeon will know that the wrong implant was selected.

In additional or alternative embodiments, the process of displaying the virtual model involves a system check prior to a surgical operation. For example, displaying the virtual model includes verifying that the cameras are tracking an instrument and the XR headset properly, that the XR headset is properly positioned on the surgeon's head, and that the virtual content is being properly rendered.

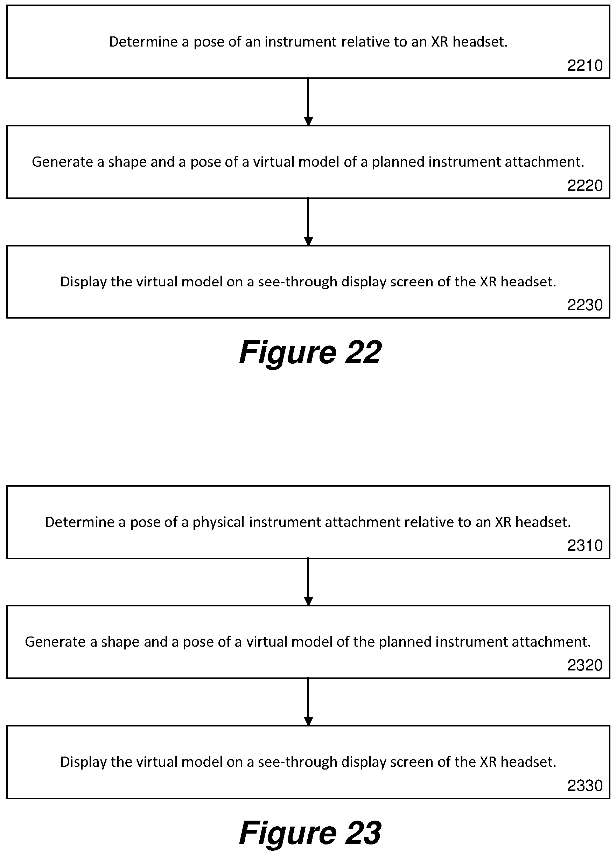

Figure 22 is a flow chart of a process performed by a surgical system (e.g., surgical system 900). At block 2210, a camera tracking system 6, 6' determines a pose of an instrument relative to an XR headset.

At block 2220, an XR headset controller 1430 generates a shape and a pose of a virtual model of a planned instrument attachment. In some embodiments, the XR headset controller 1430 generates the shape and the pose of the virtual model based on predetermined information associated with the planned instrument attachment and/or based on a pose of the instrument relative to the XR headset. The predetermined information can include characteristics of the planned instrument attachment including at least one of a size and shape. In additional or alternative embodiments, the planned instrument attachment is an implant (e.g., a pedicle screw or an expandable cage). The XR headset controller 1430 generates the virtual model of the planned instrument attachment to have graphical features representing the implant. In additional or alternative embodiments, the planned instrument attachment is an instrument extender. The XR headset controller 1430 generates the virtual model of the planned instrument attachment to have graphical features representing the instrument extender. In additional or alternative embodiments, the XR headset controller 1430 generates the virtual model to include virtual text indicating a characteristic of the planned instrument attachment.

In some embodiments, the XR headset controller 1430 determines the pose of the virtual model of the planned instrument attachment to appear attached to a location on the instrument when viewed through the see-through display. The real-world scene can include a physical instrument attachment. In some embodiments, the physical instrument attachment is attached to the location on the instrument.

In additional or alternative embodiments, the XR headset controller 1430 generates the virtual model of the planned instrument attachment to have partially transparent graphical features that at least one of which is posed to substantially align with at least one corresponding feature of the physical instrument attachment when the virtual model of the planned instrument attachment is overlaid on the physical instrument attachment while viewed through the see-through display.

In additional or alternative embodiments, the XR headset controller 1430 generates the virtual model of the planned instrument attachment to have opaque graphical features that at least one of which is posed to substantially align with at least one corresponding feature of the physical instrument attachment when the virtual model of the planned instrument attachment is overlaid on the physical instrument attachment while viewed through the see-through display.

In additional or alternative embodiments, the XR headset controller 1430 generates the virtual model of the planned instrument attachment to have opaque graphical features that at least one of which is posed to substantially align with at least one corresponding feature of the physical instrument attachment when the virtual model of the planned instrument attachment is positioned behind the physical instrument attachment while viewed through the see-through display.

In additional or alternative embodiments, the XR headset controller 1430 selectively renders graphical features of the virtual model of the planned instrument attachment as one of partially transparent and opaque based on user input. In additional or alternative embodiments, the XR headset controller 1430 selectively determines the pose of the virtual model of the planned instrument attachment to be displayed as one of an overlay on the physical instrument attachment, as behind the physical instrument attachment, and apart from the physical instrument attachment based on user input.

In additional or alternative embodiments, the XR headset controller 1430 scales the shape of the virtual model of the planned instrument attachment based on a distance determined from the pose of the physical instrument attachment relative to the XR headset.

At block 2230, XR headset 920 displays the virtual model of the planned instrument attachment on a see-through display screen of the XR headset. In some embodiments, the XR headset 920 is configured to be worn by a user during a surgical procedure and the see-through display allows at least a portion a real-world scene to pass therethrough for viewing by the user. The real-world scene can include an instrument and/or a physical instrument attachment.

In some embodiments, displaying the virtual model as partially transparent and overlaid on the physical instrument attachment allows a user to determine whether the physical instrument attachment is different than the planned instrument attachment based on the virtual model of the planned instrument attachment and the physical instrument attachment being viewable simultaneously.

In additional or alternative embodiments, displaying the virtual model as opaque and overlaid on the physical instrument attachment allows a user to determine whether the physical instrument attachment is different than the planned instrument attachment based on whether a portion of the physical instrument attachment is visible to the user.

In additional or alternative embodiments, displaying the virtual model as opaque and behind the physical instrument attachment allows a user to determine whether the physical instrument attachment is different than the planned instrument attachment based on whether a portion of the virtual model is visible to the user.

Various operations from the flow chart of Figure 22 may be optional with respect to some embodiments of surgical systems and related methods.

Figure 23 is a flow chart of a process performed by a surgical system (e.g., surgical system 900). At block 2210, a camera tracking system 6, 6' determines a pose of a physical instrument attachment relative to an XR headset. In some embodiments, the physical instrument attachment is an implant (e.g., a pedicle screw or an expandable cage). In additional or alternative embodiments, the physical instrument attachment is an instrument extender. In additional or alternative embodiments, the camera tracking system 6, 6' determines a pose of an instrument relative to the XR headset. The physical instrument attachment can be attached to a location on the instrument.

At block 2220, an XR headset controller 1430 generates a shape and a pose of a virtual model of a planned instrument attachment. In some embodiments, the shape and the pose of the virtual model of the planned instrument attachment is based on predetermined information of the planned instrument attachment and based on a pose of the physical instrument attachment relative to the XR headset. In additional or alternative embodiments, the XR headset controller 1430 generates the pose of the virtual model of the planned instrument attachment at a predetermined pose relative to the pose of the physical instrument attachment. In additional or alternative embodiments, the XR headset controller 1430 adjust an orientation of the virtual model of the planned instrument attachment relative to the XR headset to match an orientation of the physical instrument attachment. In additional or alternative embodiments, the XR headset controller scales the shape of the virtual model of the planned instrument attachment based on a distance determined from the pose of the physical instrument attachment relative to the XR headset.

In some embodiments, the XR headset controller selectively determines the pose of the virtual model of the planned instrument attachment to be displayed as one of an overlay on the physical instrument attachment, as behind the physical instrument attachment, and apart from the physical instrument attachment based on user input. In additional or alternative embodiments, the XR headset controller generates the virtual model to include virtual text indicating a characteristic of the planned instrument attachment based on the predetermined information associated with the planned instrument attachment.

At block 2230, XR headset 920 displays the virtual model of the planned instrument attachment on a see-through display screen of the XR headset. In some embodiments, the XR headset is configured to be worn by a user during a surgical procedure and the see-through display screen allows at least a portion of a real-world scene to pass therethrough for viewing by the user. The real-world scene can include the physical instrument attachment and/or an instrument.

Various operations from the flow chart of Figure 22 may be optional with respect to some embodiments of surgical systems and related methods.

### Further Definitions and Embodiments:

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

## Claims

1. A surgical system comprising:
- an instrument (1602) having trackable markers (1630, 1632);
- a physical instrument attachment (1820) configured to be attached to the instrument (1602);
- a camera tracking system (1440) configured to track the instrument (1602) via the trackable markers (1630, 1632);
- an extended reality ("XR") headset (920) configured to be worn by a user during a surgical procedure and including a see-through display screen (1302) configured to display a virtual model (1830) of a planned instrument attachment and to allow at least a portion of a real-world scene to pass therethrough for viewing by the user, the real-world scene including the instrument (1602); and
- an XR headset controller (1430) configured to generate a shape and a pose of the virtual model (1830) of the planned instrument attachment based on predetermined information associated with the planned instrument attachment and based on a pose of the instrument (1602) relative to the XR headset (920), **characterized in that**:
- the XR headset controller (1430) being further configured to selectively determine the pose of the virtual model (1830) of the planned instrument attachment to be displayed as one of an overlay on the physical instrument attachment (1820), as behind the physical instrument attachment (1820), and apart from the physical instrument attachment (1820) based on user input and wherein the XR headset controller (1430) is further configured to scale the shape of the virtual model (1830) of the planned instrument attachment based on a distance determined from the pose of the physical instrument attachment (1820) relative to the XR headset (920).

2. The surgical system of Claim 1, wherein the XR headset controller (1430) is further configured to determine the pose of the virtual model (1830) for the planned instrument attachment to appear attached to a location on the instrument (1602) when viewed through the see-through display screen (1302).

3. The surgical system of Claim 2, wherein a real-world scene further includes the physical instrument attachment attached to the location on the instrument (1602).

4. The surgical system of Claim 3, wherein the XR headset controller (1430) is further configured to generate the virtual model (1830) of the planned instrument attachment to have partially transparent graphical features that at least one of which is posed to substantially align with at least one corresponding feature of the physical instrument attachment (1820) when the virtual model (1830) of the planned instrument attachment is overlaid on the physical instrument attachment (1820) while viewed through the see-through display.

5. The surgical system of Claim 1, wherein the XR headset controller (1430) is further configured to generate the virtual model (1830) of the planned instrument attachment to have opaque graphical features that at least one of which is posed to substantially align with at least one corresponding feature of a physical instrument attachment (1820) when the virtual model (1830) of the planned instrument attachment is overlaid on the physical instrument attachment (1820) while viewed through the see-through display (1302)

6. The surgical system of Claim 1, wherein the XR headset controller (1430) is further configured to generate the virtual model (1830) of the planned instrument attachment to have opaque graphical features that at least one of which is posed to substantially align with at least one corresponding feature of the physical instrument attachment (1820) when the virtual model (1830) of the planned instrument attachment is positioned behind the physical instrument attachment (1820) while viewed through the see-through display (1302).

7. The surgical system of Claim 1, wherein the XR headset controller (1430) is further configured to selectively render graphical features of the virtual model (1830) of the planned instrument attachment as one of partially transparent and opaque based on user input

8. The surgical system of Claim 1, wherein a real-world scene further includes a physical instrument attachment (1820), and wherein
- the XR headset controller (1430) is further configured to generate the virtual model (1830) of the planned instrument attachment to have graphical features representing at least one of a pedicle screw or an expandable cage.

9. The surgical system of Claim 1, wherein the XR headset controller (1430) is further configured to generate the virtual model (1830) of the planned instrument attachment to have graphical features representing an instrument extender.

10. The surgical system of Claim 1, wherein the XR headset controller (1430) is further configured to generate the virtual model (1830) to include virtual text indicating a characteristic of the planned instrument attachment, based on the predetermined information associated with the planned instrument attachment.

11. The surgical system of Claim 1, further comprising:
- a tracking system (6, 6') configured to determine the pose of the instrument relative to the XR headset (920);
- a surgical robot (4) that includes:
a robot base (10),
a robot arm (20) connected to the robot base (10) and configured to position an end effector (26) which is configured to guide movement of the instrument (1602),
at least one motor (850, 851, 852, 853) operatively connected to move the robot arm (20) relative to the robot base (10); and
a navigation controller (828) configured to:
- determine a target pose for the instrument (1602) based on a surgical plan defining where a surgical procedure is to be performed using the instrument (1602) on an anatomical structure and defining the planned instrument attachment to be used during the surgical procedure, and
generate steering information based on the target pose for the instrument (1602) and the pose of the instrument (1602), the steering information indicating where the instrument (1602) is to be moved under control of the at least one motor (850, 851, 852, 853).

## Patentansprüche

1. Chirurgisches System, umfassend:
- ein Instrument (1602) mit nachverfolgbaren Markern (1630, 1632),
- eine physische Instrumentenbefestigung (1820), die eingerichtet ist, um am Instrument (1602) befestigt zu werden;
- ein Kamera-Tracking-System (1440), das eingerichtet ist, um das Instrument (1602) über die nachverfolgbaren Marker (1630, 1632) nachzuverfolgen,
- ein Extended-Reality- ("XR"-) Headset (920), das eingerichtet ist, um von einem Benutzer während eines chirurgischen Eingriffs getragen zu werden, und einen durchsichtigen Bildschirm (1302) umfasst, der eingerichtet ist, um ein virtuelles Modell (1830) einer geplanten Instrumentenbefestigung anzuzeigen, und um zu ermöglichen, dass zumindest ein Teil einer Szene aus der realen Welt zum Betrachten durch den Benutzer hindurchtritt, wobei die Szene aus der realen Welt das Instrument (1602) umfasst, und
- eine XR-Headset-Steuerung (1430), die eingerichtet ist, um eine Form und eine Lage des virtuellen Modells (1830) der geplanten Instrumentenbefestigung auf der Grundlage vorbestimmter Informationen, die der geplanten Instrumentenbefestigung zugeordnet sind, und auf der Grundlage einer Lage des Instruments (1602) relativ zum XR-Headset (920) zu erzeugen, **dadurch gekennzeichnet, dass**:
- die XR-Headset-Steuerung (1430) ferner eingerichtet ist, um selektiv die Lage des virtuellen Modells (1830) der geplanten Instrumentenbefestigung zu bestimmen, um als Überlagerung über dem physischen Instrumentenaufsatz (1820), hinter dem physischen Instrumentenaufsatz (1820) oder getrennt vom physischen Instrumentenaufsatz (1820) auf der Grundlage einer Benutzereingabe angezeigt zu werden, und wobei die XR-Headset-Steuerung (1430) ferner eingerichtet ist, um die Form des virtuellen Modells (1830) der geplanten Instrumentenbefestigung auf der Grundlage eines Abstands zu skalieren, der aus der Lage der physischen Instrumentenbefestigung (1820) relativ zum XR-Headset (920) bestimmt wird.

2. Chirurgisches System nach Anspruch 1, wobei die XR-Headset-Steuerung (1430) ferner eingerichtet ist, um die Lage des virtuellen Modells (1830) für die geplante Instrumentenbefestigung zu bestimmen, dass diese bei Betrachtung durch den durchsichtigen Anzeigebildschirm (1302) an einer Position des Instruments (1602) erscheint.

3. Chirurgisches System nach Anspruch 2, wobei eine Szene aus der realen Welt ferner die physische Instrumentenbefestigung umfasst, die an der Position am Instrument (1602) befestigt ist.

4. Chirurgisches System nach Anspruch 3, wobei die XR-Headset-Steuerung (1430) ferner eingerichtet ist, um das virtuelle Modell (1830) der geplanten Instrumentenbefestigung zu erzeugen, um teilweise transparente grafische Merkmale aufzuweisen, von denen zumindest eines positioniert ist, um im Wesentlichen mit zumindest einem entsprechenden Merkmal der physischen Instrumentenbefestigung (1820) ausgerichtet zu sein, wenn das virtuelle Modell (1830) der geplanten Instrumentenbefestigung bei Betrachtung durch das durchsichtige Display über die physische Instrumentenbefestigung (1820) gelegt wird.

5. Chirurgisches System nach Anspruch 1, wobei die XR-Headset-Steuerung (1430) ferner eingerichtet ist, um das virtuelle Modell (1830) der geplanten Instrumentenbefestigung zu erzeugen, um undurchsichtige grafische Merkmale aufzuweisen, von denen zumindest eines positioniert ist, um im Wesentlichen mit zumindest einem entsprechenden Merkmal einer physischen Instrumentenbefestigung (1820) ausgerichtet zu sein, wenn das virtuelle Modell (1830) der geplanten Instrumentenbefestigung bei Betrachtung durch das durchsichtige Display (1302) über die physische Instrumentenbefestigung (1820) gelegt wird.

6. Chirurgisches System nach Anspruch 1, wobei die XR-Headset-Steuerung (1430) ferner eingerichtet ist, um das virtuelle Modell (1830) des geplanten Instrumentenaufsatzes zu erzeugen, um undurchsichtige grafische Merkmale aufzuweisen, von denen zumindest eines positioniert ist, um im Wesentlichen mit zumindest einem entsprechenden Merkmal des physischen Instrumentenaufsatzes (1820) ausgerichtet zu sein, wenn das virtuelle Modell (1830) der geplanten Instrumentenbefestigung hinter der physischen Instrumentenbefestigung (1820) bei Betrachtung durch das durchsichtige Display (1302) positioniert ist.

7. Chirurgisches System nach Anspruch 1, wobei die XR-Headset-Steuerung (1430) ferner eingerichtet ist, um grafische Merkmale des virtuellen Modells (1830) der geplanten Instrumentenbefestigung wahlweise als teilweise transparent oder undurchsichtig auf der Grundlage von Benutzereingaben darzustellen.

8. Chirurgisches System nach Anspruch 1, wobei eine Szene aus der realen Welt ferner eine physische Instrumentenbefestigung (1820) umfasst, und wobei
- die XR-Headset-Steuerung (1430) ferner eingerichtet ist, um das virtuelle Modell (1830) der geplanten Instrumentenbefestigung zu erzeugen, um grafische Merkmale aufzuweisen, die zumindest eine Pedikelschraube oder einen expandierbaren Käfig darstellen.

9. Chirurgisches System nach Anspruch 1, wobei die XR-Headset-Steuerung (1430) ferner eingerichtet ist, um das virtuelle Modell (1830) der geplanten Instrumentenbefestigung zu erzeugen, um grafische Merkmale aufzuweisen, die eine Instrumentenverlängerung darstellen.

10. Chirurgisches System nach Anspruch 1, wobei die XR-Headset-Steuerung (1430) ferner eingerichtet ist, um das virtuelle Modell (1830) zu erzeugen, um einen virtuellen Text zu umfassen, der eine Eigenschaft der geplanten Instrumentenbefestigung auf der Grundlage der vorbestimmten Informationen angibt, die der geplanten Instrumentenbefestigung zugeordnet sind.

11. Chirurgisches System nach Anspruch 1, das ferner aufweist:
- ein Tracking-System (6, 6'), das eingerichtet ist, um die Lage des Instruments relativ zum XR-Headset (920) zu bestimmen;
- einen chirurgischen Roboter (4), der umfasst:
- eine Roboterbasis (10),
- einen Roboterarm (20), der mit der Roboterbasis (10) verbunden ist und eingerichtet ist, um einen Endeffektor (26) zu positionieren, der eingerichtet ist, um eine Bewegung des Instruments (1602) zu führen,
- zumindest einen Motor (850, 851, 852, 853), der wirkverbunden ist, um den Roboterarm (20) relativ zur Roboterbasis (10) zu bewegen; und
- eine Navigationssteuerung (828), die eingerichtet ist, um:
- eine Zielposition für das Instrument (1602) auf der Grundlage eines Operationsplans zu bestimmen, der festlegt, wo ein chirurgischer Eingriff unter Verwendung des Instruments (1602) an einer anatomischen Struktur durchgeführt werden soll, und die geplante Instrumentenbefestigung festlegt, die während des chirurgischen Eingriffs verwendet werden soll, und
- Steuerungsinformationen auf der Grundlage der Zielposition des Instruments (1602) und der Position des Instruments (1602) zu erzeugen, wobei die Steuerungsinformationen angeben, wohin das Instrument (1602) bei Steuerung zumindest eines Motors (850, 851, 852, 853) bewegt werden soll.

## Revendications

1. Système chirurgical comprenant :
- un instrument (1602) comportant des marqueurs de suivi (1630, 1632) ;
- un élément de fixation physique d'instrument (1820) destiné à être fixé à l'instrument (1602) ;
- un système de suivi par caméra (1440) configuré pour suivre l'instrument (1602) à l'aide des marqueurs de suivi (1630, 1632) ;
- un casque de réalité étendue (« XR ») (920) configuré pour être porté par un utilisateur pendant une intervention chirurgicale et comprenant un écran d'affichage transparent (1302) configuré pour afficher un modèle virtuel (1830) d'un élément de fixation prévu d'instrument et pour permettre à au moins une partie d'une scène réelle de passer à travers celui-ci pour être vue par l'utilisateur, la scène réelle comprenant l'instrument (1602) ; et
- un contrôleur de casque XR (1430) configuré pour générer une forme et une pose du modèle virtuel (1830) de l'élément de fixation prévu d'instrument sur la base d'informations prédéterminées associées à l'élément de fixation prévu d'instrument et sur la base d'une pose de l'instrument (1602) par rapport au casque XR (920), **caractérisé en ce que** :
- le contrôleur de casque XR (1430) étant en outre configuré pour déterminer de manière sélective la pose du modèle virtuel (1830) de l'élément de fixation prévu d'instrument, qui doit être affiché soit sous la forme d'une superposition sur l'élément de fixation physique d'instrument (1820), soit derrière l'élément de fixation physique d'instrument (1820), soit à l'écart de l'élément de fixation physique d'instrument (1820), en fonction de l'entrée d'utilisateur et
dans lequel le contrôleur de casque XR (1430) est en outre configuré pour mettre à l'échelle la forme du modèle virtuel (1830) de l'élément de fixation prévu d'instrument, en fonction d'une distance déterminée à partir de la pose de l'élément de fixation physique d'instrument (1820) par rapport au casque XR (920).

2. Système chirurgical selon la revendication 1, dans lequel le contrôleur de casque XR (1430) est en outre configuré pour déterminer la pose du modèle virtuel (1830) de telle sorte que l'élément de fixation prévu d'instrument apparaisse comme fixé à un emplacement de l'instrument (1602) lorsqu'il est visualisé à travers l'écran d'affichage transparent (1302).

3. Système chirurgical selon la revendication 2, dans lequel une scène réelle comprend en outre l'élément de fixation physique d'instrument fixé à l'emplacement de l'instrument (1602).

4. Système chirurgical selon la revendication 3, dans lequel le contrôleur de casque XR (1430) est en outre configuré pour générer le modèle virtuel (1830) de l'élément de fixation prévu d'instrument de manière à ce qu'il comporte des caractéristiques graphiques partiellement transparentes, dont au moins une est posée de manière à s'aligner sensiblement avec au moins une caractéristique correspondante de l'élément de fixation physique d'instrument (1820) lorsque le modèle virtuel (1830) de l'élément de fixation prévu d'instrument est superposé à l'élément de fixation physique d'instrument (1820) tel qu'il est vu à travers l'écran d'affichage transparent.

5. Système chirurgical selon la revendication 1, dans lequel le contrôleur de casque XR (1430) est en outre configuré pour générer le modèle virtuel (1830) de l'élément de fixation prévu d'instrument de manière à ce qu'il présente des caractéristiques graphiques opaques, dont au moins une est posée de manière à s'aligner sensiblement avec au moins une caractéristique correspondante d'un élément de fixation physique d'instrument (1820) lorsque le modèle virtuel (1830) de l'élément de fixation prévu d'instrument est superposé à l'élément de fixation physique d'instrument (1820) tel qu'il est vu à travers l'écran d'affichage transparent (1302).

6. Système chirurgical selon la revendication 1, dans lequel le contrôleur de casque XR (1430) est en outre configuré pour générer le modèle virtuel (1830) de l'élément de fixation prévu d'instrument de manière à ce qu'il présente des caractéristiques graphiques opaques dont au moins une est posée de manière à s'aligner sensiblement avec au moins une caractéristique correspondante de l'élément de fixation physique d'instrument (1820) lorsque le modèle virtuel (1830) de l'élément de fixation prévu d'instrument est positionné derrière l'élément de fixation physique d'instrument (1820) tel qu'il est vu à travers l'écran d'affichage transparent (1302).

7. Système chirurgical selon la revendication 1, dans lequel le contrôleur de casque XR (1430) est en outre configuré pour rendre de manière sélective les caractéristiques graphiques du modèle virtuel (1830) de l'élément de fixation prévu d'instrument, comme étant soit partiellement transparentes, soit opaques, en fonction de l'entrée d'utilisateur.

8. Système chirurgical selon la revendication 1, dans lequel une scène réelle comprend en outre un élément de fixation physique d'instrument (1820), et dans lequel
- le contrôleur de casque XR (1430) est en outre configuré pour générer le modèle virtuel (1830) de l'élément de fixation prévu d'instrument, de manière à ce qu'il comporte des caractéristiques graphiques représentant au moins l'un des éléments suivants : une vis pédiculaire ou une cage expansible.

9. Système chirurgical selon la revendication 1, dans lequel le contrôleur de casque XR (1430) est en outre configuré pour générer le modèle virtuel (1830) de l'élément de fixation prévu d'instrument de manière à ce qu'il comporte des caractéristiques graphiques représentant une rallonge d'instrument.

10. Système chirurgical selon la revendication 1, dans lequel le contrôleur de casque XR (1430) est en outre configuré pour générer le modèle virtuel (1830) de manière à ce qu'il comprenne un texte virtuel indiquant une caractéristique de l'élément de fixation prévu d'instrument, sur la base des informations prédéterminées associées à l'élément de fixation prévu d'instrument.

11. Système chirurgical selon la revendication 1, comprenant en outre :
- un système de suivi (6, 6') configuré pour déterminer la pose de l'instrument par rapport au casque XR (920) ;
- un robot chirurgical (4) qui comprend :
une base de robot (10),
un bras de robot (20) relié à la base de robot (10) et configuré pour positionner un effecteur terminal (26) configuré pour guider le mouvement de l'instrument (1602),
au moins un moteur (850, 851, 852, 853) connecté de manière opérationnelle pour déplacer le bras de robot (20) par rapport à la base de robot (10) ; et
un contrôleur de navigation (828) configuré pour :
- déterminer une pose cible pour l'instrument (1602) sur la base d'un plan chirurgical définissant l'emplacement où une intervention chirurgicale doit être réalisée à l'aide de l'instrument (1602) sur une structure anatomique, ainsi que l'élément de fixation prévu d'instrument qui sera utilisé au cours de l'intervention chirurgicale, et
générer des informations de pilotage en fonction de la pose cible pour l'instrument (1602) et de la pose de l'instrument (1602), les informations de pilotage indiquant où l'instrument (1602) doit être déplacé sous le contrôle de l'au moins un moteur (850, 851, 852, 853).
